(19) Europäisches Patentamt
European Patent Office
Office européen des brevets

(11) **EP 2 675 338 B1**

(12) **EUROPEAN PATENT SPECIFICATION**

(45) Date of publication and mention
of the grant of the patent:
**20.07.2016 Bulletin 2016/29**

(21) Application number: **11703622.8**

(22) Date of filing: **15.02.2011**

(51) Int Cl.:
*A61B 3/10* (2006.01)    *A61B 3/18* (2006.01)
*G01B 11/00* (2006.01)   *G01N 21/47* (2006.01)
*G01N 21/41* (2006.01)

(86) International application number:
**PCT/EP2011/000711**

(87) International publication number:
**WO 2012/110051 (23.08.2012 Gazette 2012/34)**

(54) **SYSTEM AND METHOD FOR MEASURING INTERNAL DIMENSIONS OF AN OBJECT BY OPTICAL COHERENCE TOMOGRAPHY**

SYSTEM UND VERFAHREN ZUR MESSUNG DER INNEREN ABMESSUNGEN EINES GEGENSTANDES MITTELS OPTISCHER KOHÄRENZTOMOGRAFIE

SYSTÈME ET PROCÉDÉ POUR MESURER LES DIMENSIONS INTERNES D'UN OBJET PAR TOMOGRAPHIE PAR COHÉRENCE OPTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.12.2013 Bulletin 2013/52**

(73) Proprietor: **WaveLight GmbH**
**91058 Erlangen (DE)**

(72) Inventors:
• **VOGLER, Klaus**
**99444 Blankenhain (DE)**
• **WUELLNER, Christian**
**91094 Bräuningshof (DE)**
• **GORSCHBOTH, Claudia**
**90411 Nürnberg (DE)**

• **DONITZKY, Christof**
**90542 Eckental/Eschenau (DE)**

(74) Representative: **Frenkel, Matthias Alexander**
**Wuesthoff & Wuesthoff**
**Patentanwälte PartG mbB**
**Schweigerstrasse 2**
**81541 München (DE)**

(56) References cited:
**EP-A1- 1 666 838         EP-A1- 2 020 205**
**WO-A1-2004/002298   WO-A1-2009/061756**
**WO-A1-2010/074279   WO-A1-2010/119913**
**WO-A1-2010/128626   WO-A1-2010/134564**
**WO-A1-2011/050249   DE-A1-102005 058 220**
**DE-B3-102007 023 293  US-A1- 2007 076 217**
**US-A1- 2007 076 223   US-A1- 2008 140 325**
**US-A1- 2008 285 043**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a system and a method for optically measuring internal dimensions of an object by means of optical coherence tomography, wherein said object comprises internal interfaces at which the (optical) refraction index changes, so that a portion of incident light is backreflected and/or backscattered and can be detected. The object can generally be any sample object that is at least partially transparent in an at least internal partial volume for wavelengths in a wavelength range of operating wavelength used by an optical coherence tomography (OCT) device for measuring internal dimensions in said volume. The object may comprise relatively complex external and internal structures associated with refraction index changes, and may for example be an object made of transparent plastics having complex internal structures made from modifications of the plastics associated with differing refraction indices, or samples of biological tissue, such as an eye, in particular a human eye.

Applications of optical coherence tomography (OCT) to the characterization of geometrical and optical characteristics of notably human eyes are known, e.g. in the diagnosis of the eye, when measuring the geometrical and optical characteristics of different sections of the eye and of the eye as a whole which are relevant to obtain a model of the individual eye of a patient as a basis for developing an optimum treatment plan for the patient's eye for refractive surgery including e.g. laser-based refraction corrections. At present, different diagnosis devices based on different measurement principles must be involved to obtain a precise diagnostic of the geometrical and optical characteristics of different sections of the eye, including e.g. the corneal and anterior segment (CAS) of the eye, and of the eye as a whole including the eye's length and the geometric structure of rear portions of the eye including the retina. The precision required, i.e. an axial resolution $\Delta z$ and a lateral resolution $\Delta x$, is different for the afore-mentioned sections of the eye. For example, the axial resolution obtained with conventional devices in characterizing the topography and thickness of the CAS is between approximately 5 and 10 $\mu$m, while a precision resp. measurement accuracy or resolution of less than 3 $\mu$m, preferably less than 1 $\mu$m, would be desirable for an optimum planning and *a priori* calculation of a refractive correction treatment. On the other hand, the length, notably the axial length, of the eye and positions of major refraction index interfaces distributed along the length require only an accuracy resp. resolution $\Delta z$ of approximately 50 $\mu$m or better. Conventionally, treatment plannings in (optical) refractive surgery of an eye are based on individual measurements with different diagnosis devices, which may use different measurement and evaluation principles. This renders problems when integrating the measurement data obtained from the different devices into a single model of an individual eye and attempting establishing a single integrated treatment, e.g. refractive surgery, planning. Also, the use of different diagnosis devices is time consuming because the devices are used sequentially and may require device-specific adjustments between the device and the eye to be characterized.

As an example, conventional treatment plannings in refractive surgery of the eye may use different diagnosis devices manufactured by the applicant, which include the so-called Allegro Topolyzer (Trademark) which is used to obtain cornea topography, notably of the cornea front surface and registration of post chamber surfaces (PCS), iris, pupil, limbus and apex; the Allegro Oculyzer (Trademark) for obtaining the topography of front and rear surfaces of the cornea, the thickness of the cornea as well as some geometric data of the anterior chamber of the eye (e.g. anterior chamber depth); Allegro Analyzer (Trademark) for obtaining integral wavefront data and perturbations of the eye as a whole resulting from individual aberrations of e.g. the cornea, lens and vitreous body as well as for obtaining a registration of the iris, pupil, limbus and blood vessels; the Allegro Biograph for determining the thickness of the cornea, the axial length of the eye as a whole and the length resp. thicknesses of further sections and elements of the eye including e.g. the anterior chamber and the lens as well as a registration of the pupil, apex, iris, limbus and blood vessels; and the Pachymeter for local, i.e. pointwise, measurement of the thickness in the center of the cornea and determining the depth of cuts and flap thickness e.g. in Laser in-situ Kerato-milieusis (LASIK). Comparable devices with corresponding properties and limitations are produced by other manufacturers and used in state of the art diagnostics of, and (refractive correction) treatment planning for, the human eye.

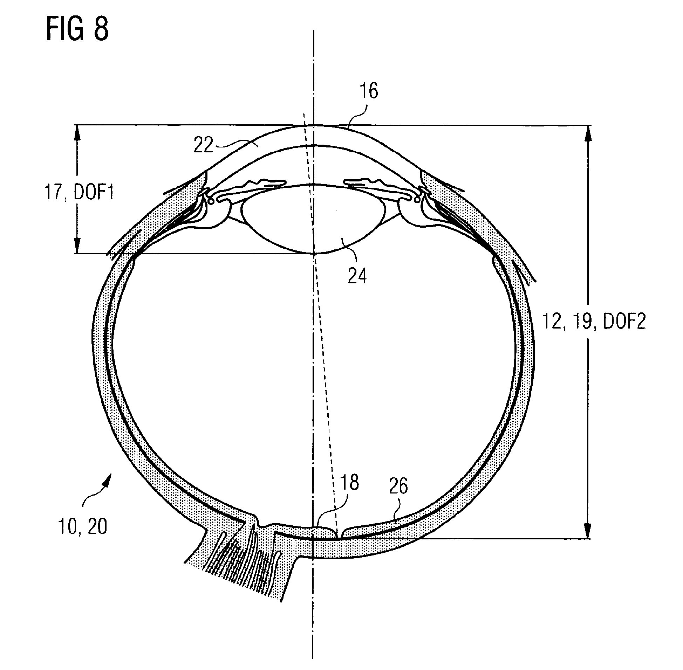

Conventional diagnosis devices aiming to precisely measure the frontal section of the eye, including the cornea, anterior chamber, iris, post chamber and the frontal surface of the lens (see Fig. 8) with the required accuracy resp. resolution are not capable to also measure e.g. the total length of the eye and the topography resp. geometry of the rear surface of the lens, while the latter data are required to calculate the total refraction of the eye. Conventionally, the data required to calculate the total refraction of the eye are determined iteratively by calculation on the basis of general model of the eye, whereby calculated data are compared with measured characteristics of wavefronts that have propagated in and through the whole eye.

An example of a high quality in vivo imaging OCT device for anterior segment imaging is disclosed in the article "Anterior segment imaging with Spectral OCT system using a high-speed CMOS camera" by I. Grulkowski et al., published on 12 March 2009 in OPTICS EXPRESS 4842, Vol. 17, No. 6. Another example is disclosed in the article "Extended in vivo anterior eye-segment imaging with full-range complex spectral domain optical coherence tomography" by J. Jungwirth et al., published in the Journal of Biomedical Optics Letters, Vol. 14(5), September/October 2009. A further example of a measurement of the anterior segment is a device CASIA SS-1000 manufactured by the company TOMEY and described in the system specifications issued therewith.

A first example of the state of the art for measuring the full axial length of an eye, as used to generate a 3D tomogram model, is disclosed in the article "Three-dimensional ophthalmic optical coherence tomography with a refraction correction algorithm", by R.J. Zawadzki et al., published in SPIE proceedings, Vol. 5140 and in the article "Iterative Berechnung von Ablationsprofilen in der Refraktiven Chirurgie" by Dr. H.P. Iseli et al., published in Augenspiegel, Vol. 20, 07-08 2008.

While the discussion of the state of the art of optical measurements of internal dimensions of a sample object comprising internal interfaces at which the refraction index changes focused on particular applications related to the diagnosis of the eye, similar constraints and limitations are also encountered in optical investigations of other types of objects as mentioned above.

In view of the afore-mentioned problems of the state of the art related to the use of different devices for obtaining the different characteristics in different internal partial volumes of an object, such as the eye, it is a general object of the present invention to save diagnosis time and cost associated with the use of various diagnosis devices, and a particular technical goal to achieve adequate accuracy of the measurement (resolution) for the different sections of the eye to enable a precise individual (customized) treatment for visual correction of a patient's eye.

The object is achieved, according to the invention, in general by providing a single system that allows to measure and obtain the different data in a practically single measurement operation, for example a single diagnostic investigation. In other words, the patient experiences (suffers) one measurement activity only even if more than one parameter is measured. The invention involves the integration of different optical coherence tomography (OCT) devices dedicated to different measurement tasks, to the measurement of different internal partial volumes of the object to be investigated with different appropriate (axial and lateral) resolution resp. accuracy. According to a first aspect of the present invention, as claimed, there is provided a system for optically measuring internal dimensions of an object comprising internal interfaces at which the refraction index changes so that a portion of incident light is backreflected and/or backscattered and can be detected, by means of optical coherence tomography (OCT), the system comprising at least one first OCT device adapted to measure internal dimensions in a first partial volume of the object. According to the invention, the system is characterized by a combination with at least one second OCT device adapted to measure internal dimensions in a second partial volume of the same object, wherein said second partial volume is at least partially different from the first partial volume.

Document WO2010134564 A1 discloses a system according to the preamble of claim 1.

The combination of a first and a second OCT device into a single system allows to measure internal dimensions in different internal volumes of the sample object with different appropriate resp. required accuracy using a single system, in a shorter time as compared to using two separate OCT devices each in a single measurement operation.

The first partial volume may be located near or at a front side of the sample object. The front side may essentially face the system. And the second partial volume may be located near or at a rear side of the object or may extend essentially from the front side to the rear side of the object. The object may for example be an eye, particularly a human eye. Measuring the internal dimensions in different partial volumes of the object, notably the eye, with a single integrated system saves time and measurement effort, and in the case of investigating an eye reduces the suffering experienced by a patient.

The first OCT device may comprise a first reference arm and a first sample arm and the second OCT device may comprise a second reference arm and a second sample arm, wherein at least a section of the first sample arm and a section of the second sample arm are directed toward the same said object. Preferably, said section of the second sample arm is at least partially superimposed spatially with said section of the first sample arm. More preferably, said section of the second sample arm and said section of the first sample arm are directed through a common lens system. Directing the first and second sample arm toward the same object, wherein both sample arms are preferably spatially superimposed and eventually directed through a common lens system, allows to measure different features of an object involving only one single mechanical adjustment of the object with respect to the claimed system.

The first OCT device may be adapted to measure the first partial volume located near or at a front side of the object, such as the corneal and anterior section (CAS) of an eye. The second OCT device may be adapted to measure a length, as measured e.g. along a depth direction, resp. the second partial volume of the object, e.g. the total length from the anterior surface of the cornea to the retina of an eye. Combining the first and second OCT device with their different measurement targets (different partial volumes to be measured) allows reducing the cost, time and measurement effort as compared to adjusting and using different measurement devices sequentially to measure the object. In addition, the combined OCT diagnostic device provides a complete data set necessary to calculate the imaging properties of the eye as a whole with suitable accuracy in one procedure ("in one shot").

The first and second OCT devices may be adapted, respectively, to emit a first and second beam each focused with a pre-determined first and second focal length, respectively, wherein the first focal length may be shorter than the second focal length. This allows to measure different target internal volumes located at different depth with respect to a front surface of the object.

The first OCT device may be adapted to emit a first beam of first radiation having wavelengths in a first wavelength range defined by a first operating wavelength and a first bandwidth, thus defining a first axial resolution. The second OCT may be adapted to emit a second beam of second radiation having wavelengths in a second wavelength range defined by a second operating wavelength and a second bandwidth, thus defining a second axial resolution. Then, the first axial resolution may be higher than the second axial resolution. Preferably, the first axial resolution may be less than 5 $\mu$m and the second axial resolution may be greater than 15 $\mu$m. More preferably, the first bandwidth may be greater than approximately 100 nm and the second bandwidth may be smaller than approximately 20 nm. Still more preferably, the first operating wavelength may be in a range from about 700 to about 1350 nm, preferably about 700 to about 900 nm, more preferably about 750 to about 850 nm, and in particular approximately 820 nm; the first bandwidth may be in the range between about 100 nm and about 200 nm. The second operating wavelength may be in a range from about 600 nm to about 1000 nm, preferably about 620 to about 750 nm, alternatively about 800 to about 1000 nm, in particular approximately 700 nm; the second bandwidth may be in the range between approximately 5 nm and 10 nm. Providing different axial resolutions in the measurement of different internal dimensions and partial volumes of the object allows to save measurement time and reduces data volumes and data volume storage requirements, where a lower high resolution over smaller dimensions is required, whereas a lower resolution over larger dimensions is sufficient, resulting in less data to be processed as compared to a system measuring in both volumes with the same high resolution.

The first OCT device may be adapted to emit a first beam of focused radiation having wavelengths in a first wavelength range defined by a first operating wavelength and a first numerical aperture, thus defining a first lateral resolution. The second OCT device may be adapted to emit a second beam of focused radiation having wavelengths in a second wavelength range having a second operating wavelength and a second numerical aperture, thus defining a second lateral resolution. Then, the first lateral resolution may be different from the second lateral resolution. Preferably, the first lateral resolution may be higher than the second lateral resolution. More preferably, the first lateral resolution is approximately 10 $\mu$m to 20 $\mu$m (and still more preferably in combination with an axial resolution of 1 $\mu$m to 3 $\mu$m) and the second lateral resolution is approximately 50 $\mu$m to 200 $\mu$m (and still more preferably in combination with an axial resolution of 10 $\mu$m to 50 $\mu$m). Providing different lateral resolutions in different beams of focused radiation allows to adapt the resolution to different application requirements and to save measurement time, data amounts and data storage requirements.

The first OCT device may be a spectral-domain OCT device and the second OCT device may be a time-domain OCT device. Alternatively, both the first OCT device and the second OCT device may be a spectral-domain OCT device. Still alternatively, both the first and the second OCT device may be a time-domain OCT device. Adapting the type of the OCT device (spectral-domain or time-domain) to the different partial volumes of the object to be investigated allows optimizing measurement accuracy, minimizing measurement time and adapting/optimizing the speed of data acquisition according to the application of investigating an object.

The first OCT device may have a first sample arm comprising a first lens system and a common lens system, wherein the first lens system and the common lens system are arranged on a first optical axis and in combination form a first focused portion of a first beam in the first sample arm, wherein the first focused beam portion has a first focal length. The second OCT device may have a second sample arm comprising a third lens system, said common lens system and a spectrally partially reflecting mirror arranged between the first lens system and the common lens system so as to direct a second beam passing along a direction of a second optical axis through the third lens system into the direction of the first optical axis and passing through said common lens system, wherein the third lens system and the common lens system in combination form a second focused portion of a second beam in the second sample arm, wherein the second focused beam portion has a second focal length. In this configuration, the first focal length may be different from the second focal length. Preferably, the first focal length is smaller than the second focal length. The focal length determines the depth range (range of measurement). Accordingly, more preferably, the second focal depth is designed so (i.e. sufficiently long) that the whole axial length of the eye can be measured by the second OCT device. Such arrangements, wherein the second sample arm emerges from a second direction along a second optical axis different from the direction of the first optical axis of the first beam, and is then re-directed into the direction of the first optical axis, and then passes through a common lens system together with the first beam, allows to design the first OCT device to be different from the second OCT device e.g. as concerns the type (spectral-domain or time-domain) of the OCT device, the axial and lateral resolution, the selection of the wavelength range of the radiation, the intensity of the radiation and the modulation in time of the radiation generated by the first and second OCT device. - In an alternative embodiment, e.g. intended for applications different from measurements of an eye, the second focal length may be smaller than the first focal length.

The first OCT device may comprise a first light source having a first operating wavelength and a first bandwidth and the second OCT device may comprise a second light source having a second operating wavelength and a second bandwidth. In this configuration, the first bandwidth may be greater than approximately 100 nm and the second bandwidth may be smaller than approximately 20 nm. Preferably, the first operating wavelength may be approximately 820 nm, the first bandwidth may be in the range between approximately 100 nm and about 250 nm (preferably between approximately 100 nm and about 200 nm), and the second operating wavelength may be approximately 700 nm and the second bandwidth may be smaller than 20 nm, and preferably in the range between about 5 nm and about 10 nm. Such spectral configuration of the first and second OCT devices allows the first partial volume to be investigated with a different axial resolution and preferably at a different operating wavelength as compared to the second partial volume.

The first OCT device may have a first sample arm and the second OCT device may have a second sample arm that is at least partially superimposed spatially on the first sample arm. The first and second sample arm may pass through a bi-focal common optical lens system comprising a first focussing portion acting in the first sample arm and having a first focal length and a second focussing portion acting in the second sample arm and having a second focal length. In this configuration, the first focal length may be smaller than the second focal length. - In a preferred first embodiment, the first focussing portion is a circular central portion of the bi-focal length system and the second focussing portion is an annular portion surrounding the first focussing portion. More preferably, the first and second focussing portions may have different spectral transmittance characteristics, each adapted to define an appropriate wavelength range as defined by a respective operating wavelength and bandwidth, according to the need of the investigation of the respective partial volumes, which may be at different distances resp. depths in the object, to which the respective focal lengths of the first and second focusing portion of the common lens system is adapted. - In an preferred alternative second embodiment, the bi-focal length system is embodied as a suitably designed Diffractive Optical Element (DOE) having at least two complementary regions, the first region being designed to render the first focal length and the second region being designed to render the second focal length.

The first OCT device and the second OCT device may comprise a common light source. This further reduces system costs and increases the degree of integration of the first and second OCT device.

The first OCT device may comprise a first reference arm and the second OCT device may comprise a second reference arm which is at least partially superimposed spatially on the first reference arm. The first reference arm may have an optical path length corresponding substantially to the optical path length of the first sample arm and may comprise a first mirror and a first reference arm lens system forming a first reference arm portion that is focused onto the first mirror. The second reference arm may have an optical path length corresponding substantially to the optical path length of the second sample arm and may comprise a second mirror, a second reference arm partially reflecting mirror arranged in the first reference arm in front of the first reference arm lens system and a second reference arm lens system arranged outside of the first reference arm and substantially between the second reference arm partially reflecting mirror and the second reference arm lens system, wherein the partially reflecting mirror re-directs a beam of light having a wavelength in a second wavelength range defined e.g. by a second operating wavelength and a second bandwidth and passing through the first reference arm lens system along a first reference arm direction into a second reference arm direction and through the second reference arm lens system, and wherein the second reference arm partially reflecting mirror and the second reference arm lens system form in combination a second reference arm portion that is focused onto the second mirror. Such configuration allows an at least partial integration resp. superposition of the first and second reference arms of, respectively, the first and second OCT device, while allowing the optical path lengths of the first and second reference arms to correspond substantially to the optical path lengths of the corresponding first and second sample arm.

In an alternative embodiment of the reference arms, the first OCT device comprises the first focussing portion being adapted to act on the first reference arm passing through a first focussing portion of a bi-focal reference arm common lens system and the second OCT device comprises a second reference arm which is at least partially superimposed spatially on the first reference arm and passes through a second focussing portion of said bi-focal reference arm common lens system, wherein the second focussing portion is adapted to act on the second reference arm. In this embodiment, the first reference arm further comprises a first mirror that is spectrally partially reflecting light having wavelengths in a first wavelength range defined e.g. by a first operating wavelength and a first bandwidth, and the second reference arm further comprises a second mirror that is spectrally reflecting light having wavelengths in a second wavelength range defined e.g. by a second operating wavelength and a second bandwidth. The focal length of the first focussing portion may be adapted such that the optical path length of the first reference arm corresponds substantially to the optical path length of the first sample arm and the focal length of the second focussing portion may be adapted such that the optical path length of the second reference arm corresponds substantially to the optical path length of the second sample arm. Preferably, the first focussing portion of the bi-focal reference arm common lens system is a circular central portion and the second focal portion is an angular portion surrounding the first focussing portion. In one configuration, the first and the second focussing portion of the bi-focal reference arm common lens system have different spectral transmission characteristics adapted to the application requirements of first and second beams targeting resp. first and second partial volumes of the object to be investigated. In an alternative configuration, a spectral filter having a selected spectral transmittance characteristic may be arranged behind the bi-focal reference arm common lens system.

According to a second aspect of the invention, as claimed, there is provided a method for optically measuring internal dimensions of an object comprising internal interfaces at which the refraction index changes so that a portion of incident light is backreflected and/or backscattered and can be detected. The object may for example be an eye.

According to the invention, the method comprises a step of measuring internal dimensions in a first partial volume of the object and internal dimensions in a second partial volume of the object by means of optical coherence tomography (OCT) in a single measurement operation, wherein the second partial volume is at least partially different from the first partial volume. This method achieves the same technical effect and advantages as the claimed system defined hereinbefore.

When performing the claimed method, a system as described above is used.

Further embodiments, advantages and technical effects of the invention may become apparent from the following detailed description of particular embodiments, which is not intended to imposing restrictions on the scope of the invention and which provided with reference to the appended drawings, in which:

Fig. 1    illustrates an embodiment of a conventional spectral-domain OCT device;

Fig. 2    illustrates an embodiment of a conventional time-domain OCT device;

Fig. 3    illustrates a first embodiment of a system in accordance with the invention, wherein a first OCT device and a second OCT device different from the first OCT device is combined by superimposing only portions of a first and second sample arm directed through a common lens system toward a same object to be investigated;

Fig. 4    illustrates a second embodiment of a system in accordance with the invention, wherein the first and second OCT device are further integrated to have a combined common sample arm;

Fig. 5    illustrates a spectral design of a system in accordance with the invention, providing radiation comprising wavelengths in a first wavelength range as defined by a first operating wavelength and a first bandwidth and radiation comprising wavelengths in a second wavelength range defined by a second operating wavelength and a second bandwidth;

Fig. 6    illustrates a third embodiment of a system in accordance with the invention, wherein both the first and second OCT device are a spectral-domain OCT devices and have partially integrated reference arms;

Fig. 7    illustrates a fourth embodiment of a system in accordance with the invention, wherein both the first and second OCT device are a spectral-domain OCT devices and wherein the design of the reference arm differs from that of the embodiment shown in Fig. 6; and

Fig. 8    is a cross-section through a human eye for illustrating the different partial volumes and internal interfaces of the eye to be investigated.

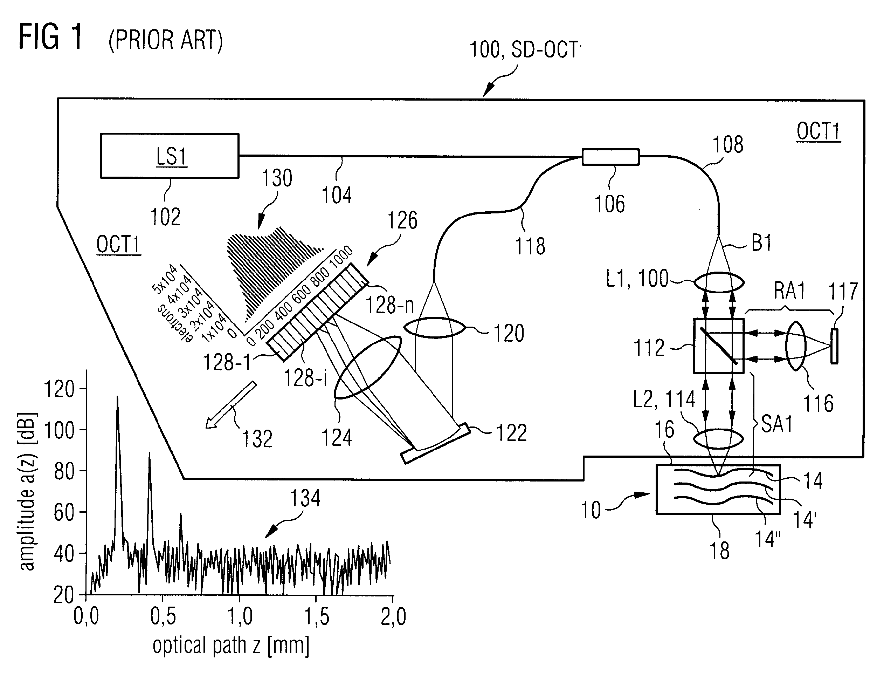

Fig. 1 shows an exemplifying conventional optical coherence tomography (OCT) device of the spectral-domain type (SD-OCT). The SD-OCT, referenced 100, comprises a preferably broadband light source 102, a light source optical fibre 104, an optical fibre coupler 106, a bi-directionally used optical fibre 108, a beam splitter 112, a sample arm comprising a first common lens system 110 (common for both sample and reference arm), a beam splitter 112 and a sample arm lens system 114, and a reference arm comprising a beam splitter 112, a reference arm lens system 116 and a reference arm mirror 117; a detection arm comprising the fibre coupler 106, a detection arm optical fibre 118, a first collimation lens system 120, an optical grating 122, a second spectrum imaging lens system 124, and a spectrometer detector array 126 comprising a plurality of detector cells 128-1 to 128-n for measuring a spectrally resolved interference pattern. The SD-OCT 100 further comprises a calculation unit 132 for performing a fast Fourier transformation of said spectrally resolved interference pattern 130 to calculate a depth distribution 134 of refractive index interfaces 14, 14', 14" in a sample object 10.

In operation of the SD-OCT 100, the light source 102 generates broadband light radiation, i.e. light radiation comprising radiation of wavelength distributed in a relatively broad spectral wavelength range. The generated radiation is transmitted through the light source optical fibre 104 via the fibre coupler 106 through the bi-directionally used optical fibre 108, from a distal end of which the radiation is emitted in the form of a divergent beam B1 passing through the first sample arm lens system 110, which reforms the beam B1 into a beam of essentially parallel light (as shown in Fig. 1) passing through the beam splitter 112. In the beam splitter 112, a portion of the beam of parallel light is transmitted into the sample arm SA1 of the SD-OCT 100 toward a second sample arm lens system 114, which focuses the beam into a focused beam portion having its focus located in the object 10.

The object 10 comprises in its internal volume a plurality of internal interfaces 14, 14', 14" at which the refraction index changes and which therefore cause partial reflections of focused beam illuminating the object 10. The radiation reflected from the plural internal interfaces 14, 14', 14" is collected by the second sample arm lens system 114, transmitted therethrough as a beam of essentially parallel light, transmits through the beam splitter 112 and is focused by the first sample arm lens system 110 into the distal end of the bi-directionally used optical fibre 108.

Another portion of the radiation transmitted from the bi-directionally used optical fibre 108 through the first sample arm lens system 110 as a beam of essentially parallel radiation is partially reflected by an internal substantially plane surface, which is oblique, preferably at an angle of substantially 45° oriented with respect to the incoming beam of essentially parallel radiation, so as to form the reference arm RA1 directed toward the reference arm lens system 116, which focuses the beam of essentially parallel radiation onto the reference arm mirror 117. The reference arm mirror 117 is arranged stationary and reflects the beam of focused radiation, so that the reflected diverging radiation is collected by the reference arm lens system 116 which transmits the reflected radiation as a beam of essentially parallel radiation from the reference arm. The radiation returning from the reference arm is directed by said plane internal surface of the beam splitter 112 toward the first sample arm lens system 110, which transmits and focuses the light returning from the reference arm RA1 onto the distal end of the bi-directionally used optical fibre 108. The optical fibre 108 thus transmits both the radiation returning from the sample arm SA1 as reflected from the internal interfaces 14, 14', 14" of the object 10 and the radiation returning from the reference arm RA1 as reflected from the reference arm mirror 117, allowing these radiation beams to interfere. The interfering radiation is transmitted through the optical fibre 108, via the fibre coupler 106 into and through the detection arm optical fibre 118, from a distal end of which the interfering radiation emerges as a diverging beam, which is collected and transmitted by the first detection lens system 120 into a beam of essentially parallel light toward the optical grating 122. The grating 122 reflects the incoming beam of interference light into a plurality of beams of essentially parallel light with different reflection angles according to the different wavelengths of the radiation impinging on the grating 122. The structure and function of the grating 122 as a spectrally resolving element reflecting impinging radiation at different reflection angles according to the wavelength of the radiation, is known to the skilled person, so that a description thereof is omitted here.

The plurality of spectrally resolved beams of radiation reflected from the grating 122 is collected by the second detection lens system 124 and focused, according to the reflection angle from the grating 122, onto the spectrometer detector array, on which the focused, spectrally resolved beams impinge on, and are detected by, respective ones of the plurality of detector cells 128-1 to 128-n.

According to this arrangement of the first detection lens 120, the optical grating 122, the second detection lens system 124 and the spectrometer detector array 126, a particular position along the spectrometer detector array 126 resp. a particular detector cell 128-i corresponds to a respective particular wavelength of the interference radiation originating from the interference of the radiation returning from the sample arm SA1 and from the reference arm RA1. The spectrometer detector array 126 thus detects the spectrally resolved interference pattern 130, which is essentially a spectral distribution of the intensity of the interference radiation. The spectral distribution is submitted to a Fourier transformation, implemented for example in the fast Fourier transformation calculation unit 132, to yield the depth distribution 134 of refractive index interfaces illustrated in Fig. 1. The distribution 134 comprises essentially a distribution of the intensity resp. amplitude a(z) of the interference radiation as a function of the length of the optical path z as measured in the sample arm SA1 for the contributions of the radiation reflected by the internal interfaces 14, 14', 14" in the object 10. As illustrated in Fig. 1, the distribution 134 comprises three peaks corresponding to the three internal interfaces 14, 14', 14" in the object 10 as depicted in Fig. 1.

In other words, the broadband spectral distribution of radiation emitted from the light source 102 interferes, after reflection from the refractive index discontinuities resp. internal interfaces 14, 14', 14" in the object 10 in the sample arm SA1, with the broadband spectral distribution of radiation reflected in the reference arm RA1. The respective interfering spectral intervals corresponding to the spectral resolution achieved by the optical grating 122 in combination with the particular detector cells 128-i, correspond to information from different depths of the internal interfaces 14, 14', 14" in the object 10. The calculated Fourier transformation of the spectrum registered by the spectrometer detector array 126 then yields information on the depth position of the interfaces along the depth direction z within the object 10.

In case the object 10 is a human eye, the refractive index differences of the different portions of the eye 20 (as illustrated in Fig. 8) results from the different refraction indices of the materials traversed by the radiation in the sample arm SA1, including air (refractive index 1.003), the film of tears (refractive index 1.3335), the epithel (refractive index 1.401) and the stroma (refractive index 1.3771). The afore-mentioned values of refractive indices of segments of a human eye 20 are taken from the specification of the afore-mentioned device manufactured by the company TOMEY.

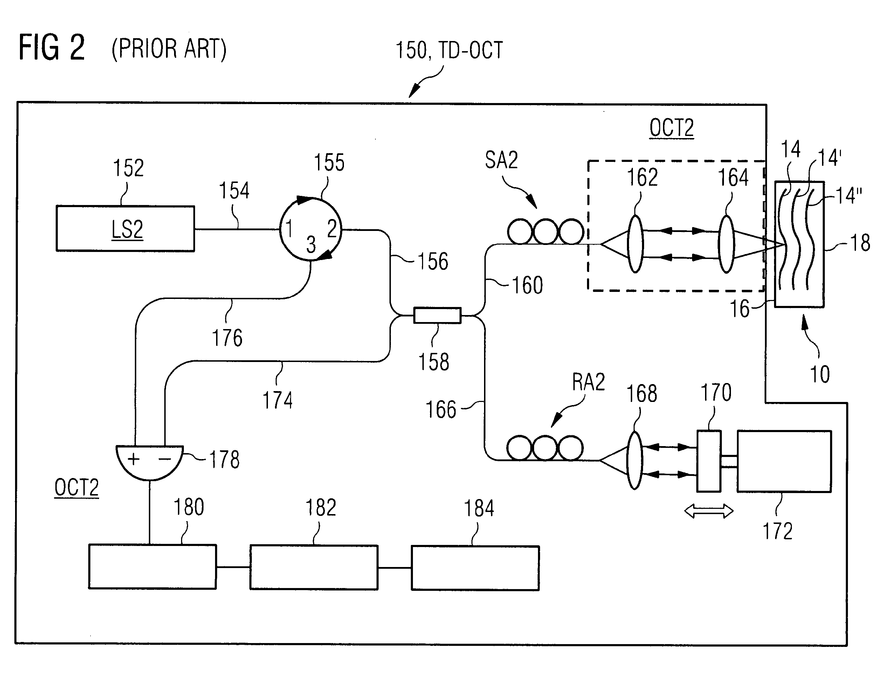

Fig. 2 depicts schematically an example of a conventional OCT device of the time-domain type (TD-OCT). The TD-OCT 150 comprises a preferably low-coherence light source 152, a first light source optical fibre 154, an optional circulator 155, a bi-directionally used second light source optical fibre 156, an optical fibre coupler 158, a sample arm SA2 comprising a bi-directionally used sample arm optical fibre 160, a first sample arm lens system 162, a second sample arm lens system 164 and a sample object 10 comprising internal interfaces 14, 14', 14" at which the refraction index changes. The TD-OCT device 150 further comprises a reference arm RA2 comprising a bi-directionally used reference arm optical fibre 166, a reference arm lens system 168, a position-modulated reference arm mirror 170 and a notably high-speed delay scanner 172. The OCT device 150 still further comprises a detection arm comprising a first detection optical fibre 174 and a detector 178. Optionally, as a means to increase the signal-to-noise ratio, the OCT device 150 further comprises the circulator 155, a second detection optical fibre 176 and a difference forming portion of the detector 178, e.g. a dual balanced signal detection (DBSD) unit. As a means for obtaining a depth information on the internal interfaces 14, 14', 14" in the object 10, the OCT device 150 still further comprises a band pass filter 180, a demodulator 182 and a computer 184 for receiving a demodulated signal and for calculating the depth information of the internal interface 14, 14', 14".

In the operation of the TD-OCT 150, the light source 152 emits radiation which suffices to be of relatively low coherence and which comprises a relatively narrow wavelength range. The radiation emitted by the light source 152 is transmitted through the first light source optical fibre 154 via the optional circulator 155, through the second light source optical fibre 156, via the optical fibre coupler 158 in which it is split into a first radiation portion propagating into the sample arm SA2 and a second radiation portion propagating into the reference arm RA2.

The first radiation portion is transmitted through the sample arm optical fibre 160, from a distal end of which it emerges as a diverging beam which is collected by the first sample arm lens system 162 transmitting the diverging beam as a beam of essentially parallel light toward the second sample arm lens system 164. The lens system 164 transmits and focuses the beam into a focused beam, the focus of which is located in the object 10. Respective internal interfaces 14, 14', 14" partially reflect portions of the incoming light back toward the second sample arm lens system 164 which collects the plurality of radiation portions reflected from the plurality of internal interfaces 14, 14', 14" and transmits these toward the first sample arm lens system 162, which focuses the reflected radiation portions returning from the sample arm SA2 onto the distal end of the sample arm optical fibre 160, which transmits this radiation via the fibre coupler 158 into the first detection optical fibre 174.

The second radiation portion split by the fiber coupler 158 is transmitted in the reference arm RA2 through the reference arm optical fibre 166, from a distal end of which it emerges as a diverging beam. This is collected by the reference arm lens system 168 and transmitted as a beam of essentially parallel radiation towards the modulated reference arm mirror 170. The reference arm mirror 170 is moved at high speed in a periodic manner to and fro along an axial direction of this portion of the reference arm RA2 by the high-speed delay scanner 172 (as indicated by the double arrow shown in Fig. 2). The radiation reflected from the position-modulated reference arm mirror 170 is transmitted and focused by the reference arm lens system 168 onto the distal end of the reference arm optical fibre 166, which transmits the reflected reference arm radiation via the fibre coupler 158 into the first detection optical fibre 174, where it interferes with the radiation returning from the sample arm SA2 as reflected by the internal interfaces 14, 14', 14" of the object 10.

The interference light is transmitted through the first detection optical fibre 174 to an input port (-) of an entrance stage of the detector 178, where a time dependency of the intensity of the interference radiation is detected and registered.

As an optional means for improving the signal-to-noise ratio and e.g. by performing a background subtraction, a portion of the radiation emitted by the light source 152 is transmitted by the circulator 155 into and through the second detection optical fibre 176 to another input port (+) of the entrance stage of the detector 178. The detector 178 subtracts from a signal from the interference radiation a signal from the radiation emitted by the light source 152 and "tapped" by the circulator 155. Due to this configuration of the detector 178 having the (+) and (-) entrance ports, excess noise from the signal of the light source 152 is subtracted from the signal of the interference radiation, thereby improving the signal-to-noise ratio. The so obtained signal is fed through the band pass filter 180 and to the demodulator 182 to remove a high-frequency component resulting from the high-speed modulation of the delay scanner 172 in the reference arm RA2. The so obtained signal is fed to, and registered in, the computer 184, which calculates from the received signal the desired depth information of the internal interfaces 14, 14', 14" in the object 10.

In the TD-OCT 150, the narrow band interference radiation is reduced by the interference of radiation reflected from the internal interfaces 14, 14', 14" in the object 10 in the sample arm SA2 with radiation returning from the reference arm RA2, the optical path length of which is scanned resp. varied by means of the periodic movement of the mirror 170 as generated by the delay scanner 172.

OCT devices of the spectral-domain type (as exemplified in Fig. 1) have as advantages over the commercially mostly used OCT devices of the time-domain type (as exemplified in Fig. 2) a better resp. higher signal-to-noise ratio and a simultaneously obtainable depth information of the internal interfaces 14, 14', 14" without involving mechanically moving parts such as the reference arm mirror 170 of the time-domain OCT device 150.

From fundamental principles of optics it can be derived, and is known to the skilled person, that the axial resolution $\Delta z$ of an OCT device, hence the accuracy for obtaining depth positions of the internal interfaces 14, 14', 14", is determined essentially by a bandwidth ($\Delta\lambda$) and a center wavelength ($\lambda_0$) of the radiation used according to:

$$\Delta z = \frac{2\ln 2}{\pi\, n} \times \frac{\lambda_o^2}{\Delta\lambda}, \qquad\qquad (1)$$

wherein $n$ is the refractive index of a medium presenting the partially reflecting interface. In case that the object 10 is a human eye 20 illustrated in Fig. 8, the relevant refractive index of the foremost interface is that of the cornea having $n$ = 1.3771.

The accuracy, by which the depth information is obtained in a lateral direction with respect to the axial direction (z), i.e. the lateral resolution $\Delta x$ is essentially determined by:

$$\Delta x = \frac{2}{\pi} \times \frac{\lambda_0}{NA} \text{ and } NA \propto \frac{1}{f}, \qquad (2)$$

wherein $NA$ is the numerical aperture of a focusing lens system, f is the focal length of the lens system which focuses the radiation in the sample arm on the object 10.

The axial range, from which a sufficiently intensive portion of radiation is reflected resp. scattered back in the object 10, is of the order of magnitude of the depth of focus (DOF) of the lens system which focuses the radiation into the object 10, and is determined by the focal length $f$ respectively the numerical aperture $NA$ of the lens system according to:

$$DOF \propto 1/NA^2 \propto f^2 \qquad (3)$$

When evaluating equations (1), (2) and (3) for an axially extending object for which the second partial volume extends substantially along the total length of the object and the first partial volume is near or at the front side 16 of the object 10 and extends from the front side over only e.g. one tenth of the total axial length, it becomes clear that an OCT device adapted to measure internal surfaces within the first partial volume with adequate accuracy cannot also measure internal interfaces distributed along the total length, i.e. the second partial volume, with the same resolution as in the first partial volume of the object 10. In particular, when the object 10 is a human eye 20 (as shown in Fig. 8), it is not possible to obtain with one single OCT device adapted to measure the frontal section of the eye including the cornea and anterior section (CAS) with high accuracy resp. axial resolution of $\Delta z = 1 \mu m$ to $3\mu m$ also the length of the eye 20 from the cornea to the retina 26.

In conventional practice, the intra-ocular structures of the CAS of an eye 20 are measured using an OCT device of the spectral-domain type having a relatively high axial resolution of less than 10 $\mu m$, where the axial resolution is in the range from about 1 $\mu m$ to 3 $\mu m$. For a precise measurement of the various interfaces of the CAS of the eye, it is possible within the scope of the disclosure and highly desirable if not necessary, to employ an SD-type OCT device of the latest state of the art, where the axial resolution is less than 1 $\mu m$.

On the other hand, the eye length is conventionally measured e.g. by devices based on the principle of optical low coherence reflectometry (OLCR) or using OCT devices of the time-domain type, wherein the length of the reference arm must be varied (scanned) over a length corresponding to the length of the eye, wherein this is achieved by axially scanning a mirror over such a length equivalent or by laterally moving a prism having a corresponding basis, as implemented e.g. in an OLCR type of device manufactured by the company Haag-Streit.

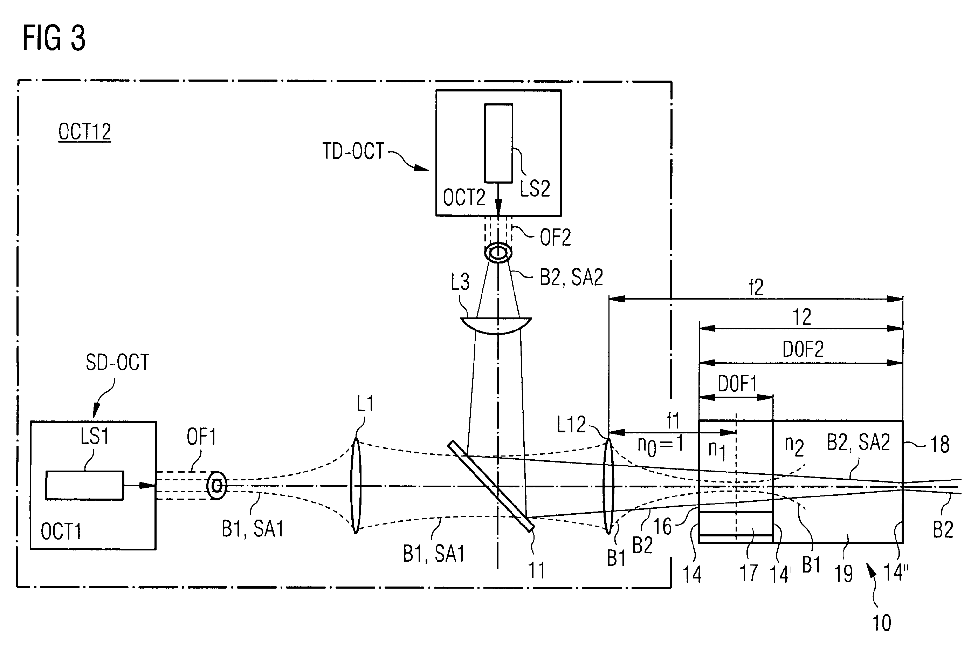

As stated above, according to the disclosure in order to enable simultaneous or quasi simultaneous measurement of both a first partial volume extending only over a relatively small portion of the total length of an object with sufficiently high resolution and a second partial volume extending e.g. along the total length or being axially spaced from the first partial volume by an axial distance of e.g. more than one half of the total length of the object, there is proposed to combine (integrate) a first and a second OCT device adapted to measure, respectively, internal dimensions in the first and the second partial volume of the object. Particular embodiments thereof are described in the following with reference to Figs. 3, 4, 6 and 7.

In the embodiments described in the following, it is assumed that the first partial volume 17 is located near or at the front side 16 of the object 10 and is measured by a first OCT device OCT1 having a focal range DOF1 extending substantially across the first partial volume 17, and that the second partial volume extends from the front side 16 to the rear side 18 of the object 10 and is measured by a second OCT device OCT2 having a corresponding depth of focus DOF2 extending thereacross.

In the first embodiment shown in Fig. 3, the first OCT device OCT1 is combined with the second OCT device OCT2 by superimposing a portion of the sample arm SA2 of the second OCT device 2 with a portion of the sample arm SA1 of the first OCT device OCT1, and by letting portions of both the sample arm SA2 of the second OCT device OCT2 and the sample arm SA1 of the first OCT device OCT1 extend through a common lens L12 and onto the same object 10. The sample arm SA1 of the first OCT device OCT1 is designed to pass through a first lens system L1 and the common lens system L12, which in combination form a focused sample arm beam portion B1 corresponding approximately to the distance of the first partial volume 17 from the common lens system L12 and a depth of focus DOF1 extending substantially throughout the first partial volume 17 of the object. The sample arm SA2 of the second OCT device OCT2 is designed to comprise a third lens system L3, a partially reflecting mirror M and the common lens system L12, whereby the third lens system L3 is arranged outside of, and the partially reflecting mirror M is arranged in, the first sample arm SA1 of the first OCT device OCT1 between the first lens system L1 and the common lens system L12, so as to deflect a portion of the sample arm SA2 of the second OCT system OCT2 into the direction of the first sample arm SA1 of the first OCT device OCT1. In particular, the portion of the sample arm SA2 of the second OCT device OCT2 is substantially perpendicular to the sample arm SA1 of the first OCT device OCT1, and the partially reflecting mirror M is arranged at an angle of substantially 45° with respect to the direction of the first sample arm SA1 of the first OCT device OCT1.

The arrangement of the partially reflecting mirror M is not limited to the aforementioned arrangement. The partially reflecting mirror M may be arranged at an angle $\theta$ different from 45°, e.g. in a range $\theta$ from 20° to 70°, and the portion of the sample arm SA2 including the third lens system L3 and the components of the second OCT device OCT2 except the sample arm lens SA2, may be arranged at an angle of $2\theta$ with respect to the sample arm SA1.

The third lens system L3 in combination with the common lens system L12 form a second focusing portion B2 having a focal length f2 corresponding substantially to a distance of a rearward half portion of the object 10 from the common lens system L12, and the depth of focus DOF2 of the second focusing portion FP2 extends substantially throughout the second partial volume 19.

The first OCT device OCT1 is a spectral-domain OCT device, e.g. of the configuration of the SD-OCT 100 shown in Fig. 1, whereby the first sample arm lens system 110 and the second sample arm lens system 114 of the SD-OCT 100 of Fig. 1 correspond, respectively, to the first lens system L1 and the common lens system L12 of the combined system shown in Fig. 3.

The second OCT system OCT2 is a time-domain OCT system, e.g. of the configuration of the TD-OCT 150 shown in Fig. 2, whereby the first sample arm lens system 162 and the second sample arm lens system 164 of the TD-OCT 150 of Fig. 2 correspond, respectively, to the third lens system L3 and the common lens system L12 of the system shown in Fig. 3, and wherein the sample arm SA2 of the device 150 of Fig. 2 is modified by "folding" the sample arm SA2 in the portion between the first and second sample arm lens systems 162 and 164 by inserting the partially reflecting mirror M as shown in Fig. 3.

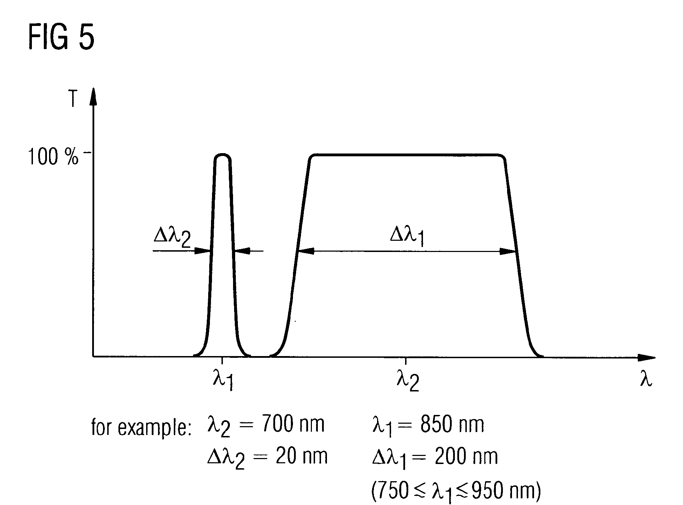

The first resp. second OCT device OCT1 resp. OCT2 has a first resp. second light source (not shown) that generate first resp. second radiation comprising respective spectra having wavelengths in a first resp. second wavelength range defined by a first resp. second operating wavelength $\lambda1$ resp. $\lambda2$ and a first bandwidth $\Delta\lambda1$ resp. $\Delta\lambda2$ as illustrated in Fig. 5.

When the first OCT device OCT1 is to be adapted to measure the CAS 22, 24 of a human eye 20 as shown in Fig. 8, a suitable first operating wavelength is $\lambda1 \approx 1.300$ nm, however, $\lambda1$ could be a wavelength in the range from about 700 nm to about 950 nm, e.g. about 850 nm as in the example of Fig. 5 (see below). The first bandwidth $\Delta\lambda1$ can be in the range from about 100 nm to about 200 nm, e.g. about 100 nm. The first OCT device OCT1 could be a spectral-domain OCT device such as the one manufactured by the company TOMEY mentioned above , wherein the light source comprises a swept-source laser having an central output wavelength $\lambda1 \approx 1310$ nm and an output power of 5 mW or less. The partially reflecting mirror M of the configuration shown in Fig. 3 can be implemented as a dichroitic mirror in a conventional way.

When the second OCT device OCT2 is to be adapted to measure the total length of a human eye 20 as shown in Fig. 8, a suitable second wavelength $\lambda2$ is in the range from about 800 nm to about 1000 nm. In the past, anterior eye OCT devices used a wavelength of about 1300 nm, but this may be changing toward shorter wavelengths because of an improved availability of suitable light detectors, which operate in the range of less than about 950 nm, such as light detectors using Si-CMOS technology. The second wavelength $\lambda2$, including the range defined by the second spectral bandwidth $\Delta\lambda2$, should be different from the first wavelength $\lambda1$ and preferably outside of range defined by the first spectral bandwidth $\Delta\lambda1$ comprising the first wavelength $\lambda1$, for example to reduce mutual cross-talk between the first and second spectral bands, and notably to ease a spectral design of a dichroitic beam splitter for splitting the first spectral band from the second spectral band (though this is not obligatory).

A preferred example of the spectral arrangement of the first and second spectral band is shown in Fig. 5. In the example of Fig. 5, the first operating wavelength $\lambda1$ is about 850 nm and the first bandwidth $\Delta\lambda1$ is 100 nm so that the first spectral band covers the range from about 750 nm to about 950 nm, which corresponds with the spectral sensitivity characteristic of Si-CMOS technology based detectors and Si-CCD detectors; the second operating wavelength $\lambda2$ is about 700 nm and the second bandwidth $\Delta\lambda2$ is considerably smaller than the first bandwidth $\Delta\lambda1$, notably less than about 20 nm. In this case, the second OCT device OCT2 can be replaced by a device based on the principle of optical low coherence reflectometry (OLCR), such as in the device manufactured by the company Haag-Streit.

The combination of the third lens system L3 and the common lens system L12 in the sample arm SA2 of the second OCT system OCT2 has a focal length f2 that is relatively long, so as to allow measuring the second partial volume 19 extending across the total axial length of the object 10 and also has a depth of focus DOF2 that is suitably designed to be relatively long so as to extend across the second partial volume 19. On the contrary, the combination of the first lens system L1 and the common lens system L12 in the first sample arm SA1 of the first OCT device OCT1 has a relatively short focal length f1 and a relatively short depth of focus DOF1, respectively, located in and extending only through the first partial volume 17 located at or near the front surface 16 of the object 10.

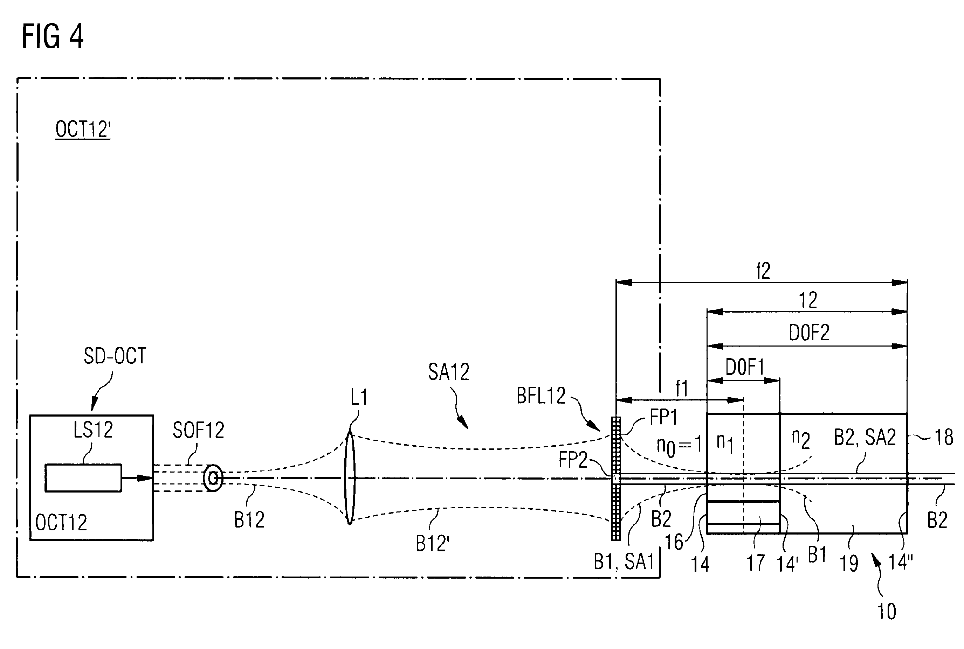

Fig. 4 illustrates a second embodiment of an integrated system OCT12', wherein the combination of the first OCT device with the second OCT device is achieved by at least partially superimposing the sample arm SA2 of the second OCT device spatially with the sample arm SA1 of the first OCT device as shown in Fig. 4, and further by integrating the first and the second OCT devices in a combined system OCT12.

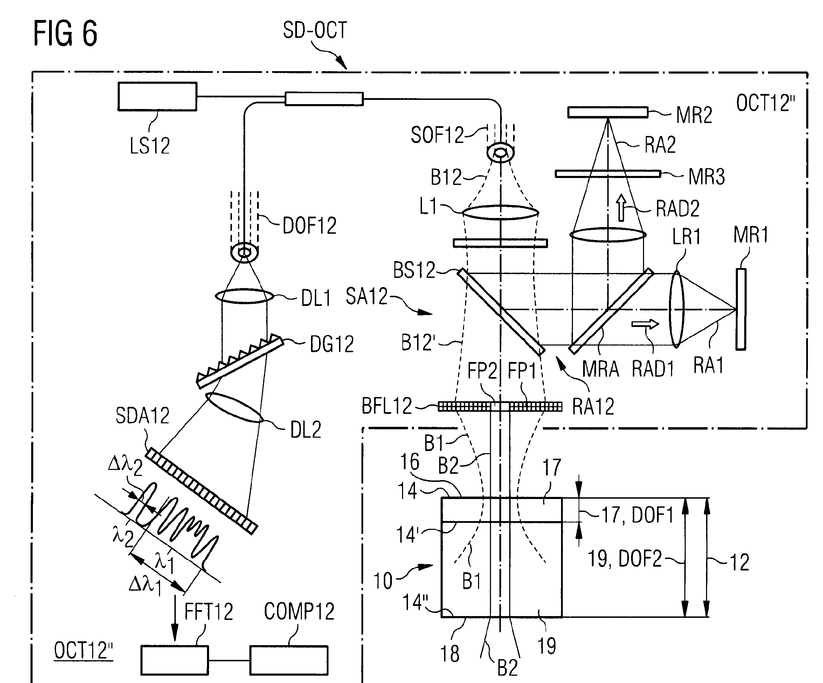

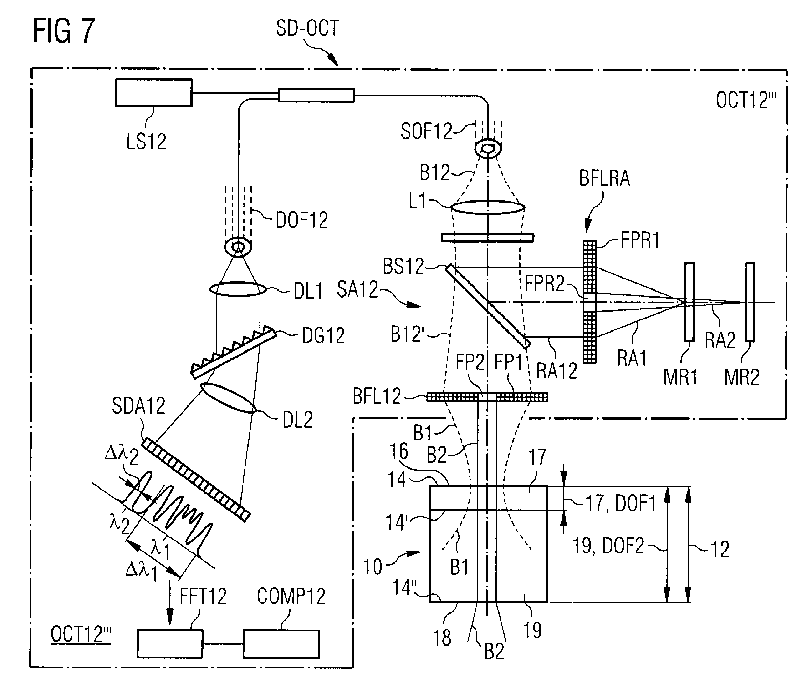

In the combined system OCT12', the detection arms of the first and the second OCT devices are shared in an integrated detection arm (not shown), and the reference arms of the first and the second OCT devices are integrated to an integrated reference arm (not shown) as implemented e.g. in the third and fourth embodiment shown respectively in Figs. 6 and 7, and further the light sources of the first and the second OCT device are integrated into a common light source LS12 of the combined system OCT12.

The system OCT12' of the second embodiment shown in Fig. 4 comprises an integration of the sample arms SA1 and SA2, respectively, of the first and second OCT device into a common sample arm, wherein a beam B1 of the first sample arm SA1 of the first OCT device and a beam B2 of the second sample arm SA2 of the second OCT device are largely spatially superimposed over each other.

As illustrated in Fig. 4, radiation associated with the first OCT device emitting at wavelengths in a first wavelength range (as shown in Fig. 5), and radiation associated with the second OCT device emitting wavelengths in a second wavelength range are guided in an integrated sample arm SA12, by guiding the radiation through a common optical fibre SOF12, from a distal end of which it emerges as a diverging common beam B12, which is collected by a first lens L1 and transmitted as a common beam B12' of essentially parallel light propagating toward a common lens system embodied as a bi-focal common lens system BFL12 and providing a first focusing portion FP1, which acts for the radiation of the first wavelength range and a second focusing portion FP2 which acts for the radiation of the second wavelength range. The first focusing portion FP1 provides a first focal length f1 and a first depth of focus DOF1 extending through the first partial volume 17, and the second focusing portion FP2 provides a second focal length f2 and a second depth of focus DOF2 extending along the second partial volume 19 of the object 10, as shown in Fig. 4.

The common lens system BFL12 is designed such that the first and second focusing portions FP1, FP2 may be arranged one beside another, e.g. in the form of two half planes. Alternatively, as shown in Fig. 4, they are arranged one surrounding the other, whereby the first focusing portion FP1 is a central circular portion and the second focusing portion FP2 is an annular portion surrounding the central circular first focusing portion.

As an alternative to embodying the common lens system as a bi-focal common lens system, the common lens system may be embodied as a bi-focal Fresnel lens or a bi-focal diffraction optical element (DOE) having two different focal lengths, e.g. having a design similar to that of a bi-focal intra-ocular lens (IOL).

In a first sub-embodiment, the radiation of the common beam B12, B12' of radiation comprises a continuous spectrum of radiation, covering both the first and the second wavelength ranges shown in Fig. 5, and the first and second focusing portions FP1 and FP2 of the lens system BFL12, respectively, have a spectrally filtering transmission characteristics adapted to provide a high transmission coefficient, preferably greater than 90 % and preferably near to or approximately 100 %, in respectively the first and second wavelength range (as defined by the first resp. second operating wavelengths $\lambda 1$, $\lambda 2$ and the first resp. second bandwidth $\Delta\lambda 1$, $\Delta\lambda 2$ as shown in Fig. 5.

In a second sub-embodiment, the spectral composition of the common beam B12, B12' in the sample arm as shown in Fig. 5 is obtained by providing respective first and second spectral filters, which are respectively congruent with the first FP1 and second FP2 focusing portions of the bi-focal common lens system BFL12.

In both the first and the second sub-embodiment of the embodiment shown in Fig. 4, upon passing through the bi-focal common lens system BFL12, the common beam B12' of radiation is divided in a first beam B1 of radiation having passed through the first focusing portion FP1 and comprising wavelength in the first wavelength range (defined by $\lambda 1$ and $\Delta\lambda 1$ of Fig. 5), and provides a first focal length f1 and a first depth of focus DOF1 extending through the first partial volume 17 of the object, and a second beam B2 of radiation having passed through the second focusing portion FP2 and comprising wavelengths in the second wavelength range (defined by $\lambda 2$ and $\Delta\lambda 2$ of Fig. 5) and provides a second focal length f2 and a second depth of focus DOF2 extending through the second partial volume 19 extending essentially through the total length 12 of the object 10. By arranging the first focal portion FP1 to surround the second focusing portion FP2 in the bi-focal common lens system BFL12, the first focusing portion FP1 has a greater diameter than the second focusing portion FP2 and accordingly the first beam B1 has a greater numerical aperture than the second beam B2. Consequently, according to equation (2), the first beam B1 in the first partial volume 17 achieves a smaller lateral resolution $\Delta x$ than the second beam B2 in the second partial volume 19. According to equation (3), the depth of focus DOF1 of the first beam B1 is smaller than the depth of focus DOF2 of the second beam B2, which is adapted to measure the second partial volume 19. In addition, since the first bandwidth $\Delta\lambda 1$ of the first beam B1 is relatively broad, e.g. in the range of about 100 nm to 200 nm, and the first operating wavelength $\lambda 1$ is in the range between about 700 nm and 950 nm, e.g. 850 nm, and the bandwidth $\Delta\lambda2$ of the second beam B2 is relatively narrow band e.g. less than about 20 nm, according to equation (1), the axial resolution $\propto 1/\Delta z1$ of the first beam B1 is considerably higher than the axial resolution $\propto 1/\Delta z2$ of the second beam B2, or stated otherwise, $\Delta z1 \ll \Delta z2$.

When both the first and the second OCT systems are SD-OCT type devices, they can have an integrated sample arm (as shown in Fig. 4, 6 or 7), can share a common light source LS12 (as shown in Figs. 6 and 7), and can further integrate their reference arms (e.g. as in the embodiments shown in Figs. 6 and 7).

Such configurations can be particularly adapted to measure the CAS section 17 and the total length 19 of a human eye 20 (see Fig. 8), when the light source LS12 is a broadband light source suitable for SD-OCT and has an emission spectrum ranging from e.g. less than about 700 nm to more than about 950 nm. This range is well adapted to the spectral sensitivity of modern high-speed silicon (SI)-based detectors. The first wavelength can be spectrally filtered out from the emission spectrum of the shared light source LF12 e.g. by a spectrally filtering element designed to transmit radiation having wavelengths around a central first operating wavelength $\lambda1$ of about 820 nm and a first bandwidth $\Delta\lambda1$ in the range 100 nm to 200 nm. Also the second wavelength range can be filtered out from the emission spectrum of the shared light source LS12 e.g. by a filter designed to transmit wavelengths around a central second operating wavelength $\lambda2$ of about 700 nm and a second bandwidth $\Delta\lambda2$ in the range of about 5 nm to about 20 nm.

Respective spectral filters can be provided separately in the integrated sample arm SA12 so as to be congruent with the first and second focussing portions FP1, FP2 of the bi-focal common lens system BFL12, or can be applied directly on the first and second focussing portions FP1, FP2 of the bi-focal common lens system BFL12, e.g. by respective suitable spectral filter coatings, notably edge filter coatings, where the edge of a first edge filter applied in the first focussing portion FP1 is designed to be positioned between the first and second wavelength ranges shown in Fig. 5.

Fig. 6 illustrates a third embodiment of a combined system OCT12". The combined system OCT12" has a configuration essentially as a spectral-domain OCT device shown in Fig. 1, however with the following modifications with respect to the embodiment shown in Fig. 4.

Firstly, the sample arm SA12 comprising a sample optical fiber SOF12, the first length system L1 and the bi-focal common lens system BFL12 is configured as in the second embodiment shown in Fig. 4. Secondly, the light source LS12 is a broadband source adapted for SD-OCT application and a spectral filtering of the broadband radiation spectrum is provided either by applying respective first and second spectral filters, on the first and second focussing portions FP1, FP2 of the bi-focal common lens system BFL12 for filtering out the first and second wavelength ranges (defined as shown in Fig. 5). Thirdly, the detector arm is integrated by using a common detection arm optical fiber DOF12 for guiding the interference radiation from the first and second beams B1 and B2, a first detection arm optical lens system DL1, a common detection arm grating DG12, a second detection arm optical lens system DL2 and a common detection arm spectrometer detector array SDA12, which in combination form a similar configuration as the first detection lens system 120, the optical grating 122, the second detection lens system 124 and the spectrometer detector array 126 of the SD-OCT device 100 shown in Fig. 1. However, the common detector arm grating DG12 in combination with the common detector arm spectrometer detector array SDA12 is adapted to detect and spectrally resolve radiation comprising both the first and second wavelength ranges as produced by the spectral filters as described above and as illustrated in Fig. 5.

Fourthly, the reference arm is integrated by at least partially superimposing spatially a first and a second reference arm RA1 and RA2 corresponding, respectively, to the first and second sample arms SA1 and SA2. The first reference arm RA1 comprises a common beam splitter BS12, a first reference arm lens LR1 and a first reference arm mirror MR1 arranged stationary and at a position (distance) with respect to the common beam splitter BS12 so that the optical path length for the radiation RAD1 in the first reference arm RA1 corresponds to the optical path length of the radiation in the first beam B1 focused into the first partial volume 17. The second reference arm RA2 comprises said common beam splitter BS12, a second reference arm partially reflecting mirror MRA, a second reference arm lens system LR2 and a second reference arm mirror MR2, wherein the mirror MRA is arranged in the optical path of the first reference arm RA1 between the common beam splitter BS12 and the first reference arm lens system LR1 and is adapted to partially reflect (deflect) radiation RAD2 comprising wavelengths in the second wavelength range (as defined by $\lambda2$ and $\Delta\lambda2$, see Fig. 5) away from the direction of the first reference arm RA1 and towards the second reference arm lens system LR2 and the second reference arm mirror MR2. Also, the second reference arm mirror MR2 is arranged stationary and at a distance with respect to the common beam splitter BS12 so that the optical path length of the second radiation RAD2 in the second reference arm RA2 corresponds to the optical path length of the radiation of the second beam B2 of the second sample arm SA2 focused into the second partial volume 19 of the object 10. The partially reflecting mirror MRA is adapted to be selectively transmissive for the wavelengths of the first radiation RAD1 in the first spectral range and selectively reflective for the wavelengths of the second radiation RAD2 in the second wavelength range.

In order to increase the signal-to-noise ratio and/or to improve the interference signal of the first beam B1 returning from the refractive index interfaces in the first partial volume 17, an additional third reference arm mirror MR3, see Fig. 6, is provided, which is designed to be partially transmissive for wavelengths in the second wavelength range, by providing a transmission coefficient in the range of about 10% to 50%, and reflective for wavelengths in the first wavelength range. The mirror MR3 is provided in the second reference arm RA2 at a position so that the optical path between the beam splitter BS12 and the mirror MR3 corresponds to the optical path between the beam splitter BS12 and the mirror MR1.

In the embodiment shown in Fig. 6,the second reference arm lens system LR2 may have the same focal length as the second focussing portion FP2 of the bi-focal common optical lens system BFL12. Accordingly, the lens system LR2 images on the second and third reference arm mirrors MR2 and MR3 similar beam diameters over a relatively long depth of focus corresponding to the depth of focus DOF2 of the second focussing portion FP2 in the sample arm SA2, so that a sufficient reference signal is obtained.

Fig. 7 shows a fourth embodiment of a combined system OCT12''', which is similar to the third embodiment of the combined system OCT12'' shown in Fig. 6 as concerns the integration of the sample arm SA12, the light source LS12 and the detection arm, and which differs only as concerns the configuration and degree of integration of the reference arm.

In Fig. 7, the integrated reference arm RA12 comprises a bi-focal common reference arm lens system BFLRA or a suitable bi-focal diffraction optical element (DOE) designed to perform in an equal manner as the bi-focal lens system BFLRA, both of which comprise a first reference arm focussing portion FPR1 and a second reference arm focussing portion FPR2 surrounding the first reference arm focussing portion FPR1. The first reference arm portion FPR1 has a focal length adapted so as to transmit and focus radiation having wavelengths in the first wavelength range (defined by $\lambda 1$ and $\Delta\lambda 1$, see Fig. 5) on a first reference arm mirror MR1 and wherein the second reference arm focussing portion FPR2 is adapted to transmit and focus radiation comprising wavelength in the second wavelength range (defined by $\lambda 2$ and $\Delta\lambda 2$, see Fig. 5) on a second reference arm mirror MR2. The first and second reference arm mirrors MR1 and MR2 are arranged at distances with respect to the common beam splitter BS12 so that the optical path length of the first reference arm RA1 generated by the first reference arm focussing portion FPR1 corresponds to the optical path length of the first beam B1 in the sample arm SA12, and the optical path length of the second reference arm RA2 produced by the second reference arm focussing portion FPR2 corresponds to the optical path length of the second beam B2 of the sample arm SA12. The bi-focal common reference arm common lens system BFLRA can thus be configured similarly as the bi-focal common lens system BFL12 in the sample arm SA12.

In the second, third and fourth embodiments of the integrated systems OCT12', OCT12'' and OCT12''' shown in Figs. 4, 6 and 7, there may be provided a bi-focal Fresnel lens system respectively instead of the bi-focal common lens system BFL12 in the sample arms SA12 and the bi-focal reference arm common lens system BFLRA in the reference arm.

Furthermore, a distortion correction for the chromatic aberration can be provided in the reference arms RA12 of these integrated systems in order to approximate the chromatic distortion of the first and second beams B1 and B2 in the first and second partial volume 17 and 19 of the object 10 and to improve the signal-to-noise ratio of the integrated system.

List of reference signs and numerals:

| | |
|---|---|
| SD-OCT | spectral-domain OCT |
| TD-OCT | time-domain OCT |
| 10 | object sample |
| 12 | length |
| 14, 14', 14'' | internal interfaces |
| 16 | front side |
| 17 | first partial volume |
| 18 | rear side |
| 19 | second partial volume |
| 20 | eye |
| 22 | corneal section |
| 24 | anterior section |
| 26 | retina |
| 100 | spectral-domain OCT device (SD-OCT) |
| 102 | light source (broadband) |
| 104 | light source optical fiber |
| 106 | fiber coupler |
| 108 | bi-directional optical fiber |
| 110 | first sample arm lens system |
| 112 | beam splitter |

| | |
|---|---|
| 114 | second sample arm lens system |
| 116 | reference arm lens system |
| 117 | reference arm mirror |
| 118 | detection arm optical fiber |
| 120 | first detection lens system |
| 122 | optical grating |
| 124 | second detection lens system |
| 126 | spectrometer detector array |
| 128-1, | ...128-i, ...128-n detector cells |
| 130 | spectrally resolved interference pattern |
| 132 | fast Fourier transformation calculation unit |
| 134 | depth distribution of refractive index interfaces |
| 150 | time-domain OCT device (TD-OCT) |
| 152 | light source (low coherence) |
| 154 | first light source optical fiber |
| 155 | circulator (optional) |
| 156 | second light source optical fiber |
| 158 | fiber coupler |
| 160 | (bi-directionally used) sample arm optical fiber |
| 162 | first sample arm lens system |
| 164 | second sample arm lens system |
| 166 | reference arm optical fiber |
| 168 | reference arm lens system |
| 170 | reference arm mirror |
| 172 | (high-speed) delay scanner |
| 174 | first detection optical fiber |
| 176 | second detection optical fiber |
| 178 | detector |
| 180 | band pass filter |
| 182 | demodulator |
| 184 | computer |
| OCT1 | first OCT device |
| LS1 | first light source |
| RA1 | first reference arm |
| LR1 | first reference arm length system |
| RAD1 | first reference arm direction |
| SA1 | first sample arm |
| B1 | first beam |
| f1 | first focal lens |
| λ1 | first operating wavelength |
| Δλ1 | first bandwidth |
| L1 | first lens system |
| MR1 | first mirror |
| OCT2 | second OCT device |
| LS2 | second light source |
| RA2 | second reference arm |
| LR2 | second reference arm length system |
| RAD2 | second reference arm direction |
| SA2 | second sample arm |
| B2 | second beam |
| f2 | second focal lens |
| λ2 | second operating wavelength |
| Δλ2 | second bandwidth |
| L2 | second lens system |
| L3 | third lens system |
| MR2 | second mirror |
| MR3 | third mirror |
| OCT12 ... OCT 12''' | integrated system |

| LS12 | common light source |
|---|---|
| L12 | common lens system |
| BFL12 | bi-focal common lens system |
| FP1 | first focussing portion |
| FP2 | second focussing portion |
| MRA | second reference arm partially reflecting mirror |
| BFLRA | bi-focal reference arm common lens system |
| FPR1 | first focusing portion of BFLRA |
| FPR2 | second focusing portion of BFLRA |
| OF1 | first optical fiber of OCT1 |
| OF2 | second optical fiber of OCT2 |
| OF12 | common (sample arm) optical fiber of OCT12' to OCT12''' |
| SA12 | common sample arm of OCT12' - OCT12''' |
| B12, B12' | common beam of sample arm radiation |
| SOF12 | sample arm optical fiber |
| DOF 1 | first depth of focus |
| DOF 2 | second depth of focus |
| DOF12 | optical fiber of detection arm |
| DL1 | first detection arm optical lens system |
| DL2 | second detection arm optical lens system |
| DG12 | common detection arm grating |
| SDA12 | common spectrometer detector array |
| FFT12 | fast Fourier transformation unit |
| COMP12 | computer |
| BS12 | common beam splitter |

**Claims**

1. System (OCT12 - OCT12''') for optically measuring internal dimensions of a sample object (10), said object being a human eye (20), said object (10) comprising internal interfaces (14, 14', 14") at which the refraction index changes so that a portion of incident light is backreflected and/or backscattered and can be detected, by means of optical coherence tomography, OCT, comprising:

   - at least one first OCT device (OCT1) adapted to measure internal dimensions in a first partial volume (17) of the object (10), and
   - at least one second OCT device (OCT2) adapted to measure internal dimensions in a second partial volume (19) of the same object (10), wherein
   - the second partial volume (19) is at least partially different from the first partial volume (17),

   wherein the first OCT device (OCT1) is adapted to emit a first beam (B1) of first radiation having wavelengths in a first wavelength range defined by a first operating wavelength ($\lambda$1) and a first bandwidth ($\Delta\lambda$1), thus defining a first axial resolution;
   wherein the second OCT device (OCT2) is adapted to emit a second beam (B2) of second radiation having wavelengths in a second wavelength range defined by a second operating wavelength ($\lambda$2) and a second bandwidth ($\Delta\lambda$2), thus defining a second axial resolution;
   wherein the following applies:

   - the first operating wavelength is greater than the second operating wavelength, and **characterised in that**
   - the first bandwidth is considerably greater than the second bandwidth, and the first axial resolution is higher than the second axial resolution.

2. System (OCT12) according to claim 1, wherein:

   the first OCT device (OCT1) comprises a first reference arm (RA1) and a first sample arm (SA1),
   the second OCT device (OCT2) comprises a second reference arm (RA2) and a second sample arm (SA2), and
   first sample arm (SA1) and the second sample arm (SA2) are spatially superimposed.

3. System (OCT12 - OCT12''') according to anyone of the preceding claims, wherein the first OCT device (OCT1) is adapted to measure a corneal (22) and anterior (24) section of said eye (20), and/or in that the second OCT device (OCT2) is adapted to measure a length, e.g. measured along a depth direction, and/or the retina (26) of said eye (20).

4. System (OCT12, OCT12') according to any one of the preceding claims, wherein the first OCT device (OCT1) is adapted to emit a first beam (B1) focused with a pre-determined first focal length (f1) and that the second OCT device (OCT2) is adapted to emit a second beam (B2) focused with a pre-determined second focal length (f2), wherein the first focal length (f1) is shorter than the second focal length (f2).

5. System (OCT12 - OCT12''') according to anyone of the preceding claims, wherein the first OCT device (OCT1) is adapted to emit a first beam (B1) of focused radiation having wavelengths in a first wavelength range having the first operating wavelength ($\lambda$1) and a first numerical aperture (NA1), thus defining a first lateral resolution ($\Delta$x1); that the second OCT device (OCT2) is adapted to emit a second beam (B2) of focused radiation having wavelengths in a second wavelength range having the second operating wavelength ($\lambda$2) and a second numerical aperture (NA2), thus defining a second lateral resolution ($\Delta$x2), and that the first lateral resolution ($\Delta$x1) is different from, preferably smaller than, the second lateral resolution ($\Delta$x2).

6. System (OCT12) according to anyone of the claims 1 to 5, wherein the first OCT device (OCT1) is a spectral-domain OCT device (SD-OCT) and the second OCT device (OCT2) is a time-domain OCT device (TD-OCT).

7. System (OCT12 - OCT12''') according to anyone of the claims 1 to 5, wherein each of the first OCT device (OCT1) and the second OCT device (OCT2) is a spectral-domain OCT device (SD-OCT).

8. System (OCT12 - OCT12''') according to anyone of the claims 1 to 7, wherein the first OCT device (OCT1) has a first sample arm (SA1) comprising a first lens system (L1) and a common lens system (L12), wherein the first lens system (L1) and the common lens system (L12) are arranged on a first optical axis and in combination form a first focused portion of a first beam (B1) in the first sample arm (SA1), the first focused portion having a first focal length (f1); in that the second OCT device (OCT2) has a second sample arm (SA2) comprising a third lens system (L3), said common lens system (L12) and a spectrally partially reflecting mirror (M) arranged between the first lens system (L1) and the common lens system (L12) so as to re-direct a second beam (B2) passing along a direction of a second optical axis through the third lens system (L3) into the direction of the first optical axis and passing through said common lens system (L12), wherein the third lens system (L3) and the common lens system (L12) in combination form a second focused portion of the second beam in the second sample arm (SA2), the second focused portion having a second focal length (f2); and in that the first focal length (f1) is different from, preferably smaller than, the second focal length (f2).

9. System (OCT12) according to claim 8, wherein the first OCT device (OCT1) comprises a first light source (LS1) having the first operating wavelength ($\lambda$1) and the first bandwidth ($\Delta\lambda$1), and the second OCT device (OCT2) comprises a second light source (LS2) having the second operating wavelength ($\lambda$2) and the second bandwidth ($\Delta\lambda$2), and in that the first bandwidth ($\Delta\lambda$1) is in the range of 100 nm to 200 nm, and the second bandwidth ($\Delta\lambda$2) is smaller than 20 nm.

10. System (OCT12', OCT12'') according to anyone of the claims 1 to 7, wherein the first OCT device (OCT1) has a first sample arm (SA1) and the second OCT device (OCT2) has a second sample arm (SA2) at least partially superimposed spatially on the first sample arm (SA1), the first and second sample arm (SA1, SA2) pass through a bi-focal common optical lens system (BFL12) comprising a first focusing portion (FP1) having a first focal length (f1) and acting in the first sample arm (SA1), and a second focusing portion (FP2) having a second focal length (f2) and acting in the second sample arm (SA2), and in that the first focal length (f1) is different from, preferably smaller than the second focal length (f2).

11. System (OCT12 - OCT12''') according to one of claims 1 or 10, wherein the first OCT device (OCT1) and the second OCT device (OCT2) comprise a common light source (LS12).

12. System (OCT12'') according to claim 10 or 11, wherein the first OCT device (OCT1) comprises a first reference arm (RA1) and the second OCT device (OCT2) comprises a second reference arm (RA2) which is at least partially superimposed spatially on the first reference arm (RA1), that the first reference arm (RA1) has an optical path length corresponding substantially to the optical path length of the first sample arm (SA1) and comprises a first mirror (MR1) and a first reference arm length system (LR1) forming a first reference arm portion that is extending along a first reference path direction (RAD1) and focused onto the first mirror (MR1), and

that the second reference arm (RA2) has an optical path length corresponding substantially to the optical path length of the second sample arm (SA2) and comprises a second mirror (MR2), a second reference arm partially reflecting mirror (MRA), which is arranged in the first reference arm (RA1) in front of the first reference arm lens system (LR1), and a second reference arm lens system (LR2), which is arranged outside of the first reference arm (RA1) and substantially between the second reference arm partially reflecting mirror (MRA) and the second reference arm lens system (LR2), wherein the partially reflecting mirror (MRA) re-directs a beam of light having a wavelengths in a second wavelength range associated with the second reference arm (RA2) and passing through the first reference arm lens system (LR1) into a second reference arm direction (RAD2) and through the second reference arm lens system (LR2), and wherein the first reference arm lens system (LR1) and the second reference arm lens system (LR2) form in combination a second reference arm portion that is focused onto the second mirror (MR2).

13. System (OCT12''') according to claim 10 or 11, wherein the first OCT device (OCT1) comprises a first reference arm (RA1) passing through a first focusing portion (FPR1) of a bi-focal reference arm common lens system (BFLRA) and the second OCT device (OCT2) comprises a second reference arm (RA2) which is at least partially superimposed spatially on the first reference arm (RA1) and passes through a second focusing portion (FPR2) of said bi-focal reference arm common lens system (BFLRA),

in that the first reference arm (RA1) further comprises a first mirror (MR1) adapted to reflect light having wavelengths in a first wavelength range defined by the first operating wavelength ($\lambda$1) and the first bandwidth ($\Delta\lambda$1);

in that the second reference arm (RA2) further comprises a second mirror (MR2) adapted to spectrally reflect light having wavelengths in a second wavelength range defined by the second operating wavelength ($\lambda$2) and the second bandwidth ($\Delta\lambda$2);

in that a focal length of the first focusing portion (FPR1) is dimensioned such that the optical path length of the first reference arm (RA1) corresponds substantially to the optical path length of the first sample arm (SA1); and

in that a focal length of the second focusing portion (FPR2) is dimensioned such that the optical path length of the second reference arm (RA2) corresponds substantially to the optical path length of the second sample arm (SA2), wherein preferably the first focusing portion (FPR1) is a circular central portion and the second focusing portion (FPR2) is an annular portion surrounding the first focusing portion (FPR1) of the bi-focal reference arm common lens system (BFLRA).

14. Method, using a system according to any one of the claims 1-13, for optically measuring internal dimensions of an object (10), for example an eye (20), said object comprising internal interfaces (14, 14', 14") at which the refraction index changes so that a portion of incident light is backreflected and/or backscattered and can be detected, comprising a step of measuring dimensions in a first partial volume (17) of the object (10) and dimensions in a second partial volume (19) of the object by means of optical coherence tomography (OCT) in a single diagnostic investigation, wherein the second partial volume (19) is at least partially different from the first partial volume (17) of the object (10).

**Patentansprüche**

1. System (OCT12 - OCT12''') zum optischen Messen von internen Abmaßen eines Probenobjekts (10), wobei das Objekt ein menschliches Auge (20) ist, wobei das Objekt (10) interne Grenzflächen (14, 14', 14") umfasst, bei denen der Brechindex sich ändert, so dass ein Teil von einfallendem Licht zurückreflektiert und/oder rückgestreut wird und mittels optischer Kohärenztomographie, OCT, erfasst werden kann, umfassend:

- zumindest eine erste OCT-Vorrichtung (OCT1), die eingerichtet ist, um innere Abmaße in einem ersten Teilvolumen (17) des Objekts (10) zu messen, und
- zumindest eine zweite OCT-Vorrichtung (OCT2), die eingerichtet ist, um innere Abmaße in einem zweiten Teilvolumen (19) desselben Objekts (10) zu messen, wobei
- das zweite Teilvolumen (19) zumindest teilweise von dem ersten Teilvolumen (17) verschieden ist,

wobei die erste OCT-Vorrichtung (OCT1) eingerichtet ist, um einen ersten Strahl (B1) einer ersten Strahlung mit Wellenlängen in einem ersten Wellenlängenbereich abzustrahlen, der durch eine erste Betriebswellenlänge (Λ1) und eine erste Bandbreite der (Δλ1) definiert ist, wodurch eine erste axiale Auflösung definiert wird;

wobei die zweite OCT-Vorrichtung (OCT2) eingerichtet ist, um einen zweiten Strahl (B2) einer zweiten Strahlung mit Wellenlängen in einem zweiten Wellenlängenbereich abzustrahlen, der durch eine zweite Betriebswellenlänge ($\lambda$2) und eine zweite Bandbreite (Δλ2) definiert ist, wodurch eine zweite axiale Auflösung definiert wird,

wobei das Folgende gilt:

- die erste Betriebswellenlänge ist größer als die zweite Betriebswellenlänge, und

**dadurch gekennzeichnet, dass**

- die erste Bandbreite wesentlich größer als die zweite Bandbreite ist, und
- die erste axiale Auflösung höher als die zweite axiale Auflösung ist.

2. System (OCT12) gemäß Anspruch 1, wobei:

die erste OCT-Vorrichtung (OCT1) einen ersten Referenzarm (RA1) und einen ersten Probenarm (SA1) umfasst, die zweite OCT-Vorrichtung (OCT2) einen zweiten Referenzarm (RA2) und einen zweiten Probenarm (SA2) umfasst, und

der erste Probenarm (SA1) und der zweite Probenarm (SA2) räumlich überlagert sind.

3. System (OCT12 - OCT12''') gemäß zumindest einem der vorangegangenen Ansprüche, wobei die erste OCT-Vorrichtung (OCT1) eingerichtet ist, um einen kornealen (22) und einen schrägen (24) Abschnitt des Auges (20) zu messen, und/oder dahingehend, dass die zweite OCT-Vorrichtung (OCT2) eingerichtet ist, um eine Länge, z.B. entlang einer Tiefenrichtung gemessen, und/oder die Retina (26) des Auges (20) zu messen.

4. System (OCT12, OCT12') gemäß zumindest einem der vorangegangenen Ansprüche, wobei die erste OCT-Vorrichtung (OCT1) eingerichtet ist, um einen ersten Strahl (B1) abzustrahlen, der mit einer vorbestimmten ersten Fokallänge (f1) fokussiert ist, und dahingehend, dass die zweite OCT-Vorrichtung (OCT2) eingerichtet ist, um einen zweiten Strahl (B2) abzustrahlen, der mit einer vorbestimmten zweiten Fokallänge (f2) fokussiert ist, wobei die erste Fokallänge (f1) kürzer als die zweite Fokallänge (f2) ist.

5. System (OCT12 - OCT12''') gemäß zumindest einem der vorangegangenen Ansprüche, wobei die erste OCT-Vorrichtung (OCT1) eingerichtet ist, um einen ersten Strahl (B1) einer fokussierten Strahlung mit Wellenlängen in einem ersten Wellenlängenbereich mit der ersten Betriebswellenlänge (A1) und einer ersten numerischen Apparatur (NA1) abzustrahlen, wodurch eine erste laterale Auflösung ($\Delta x1$) definiert wird; dahingehend, dass die zweite OCT-Vorrichtung (OCT2) eingerichtet ist, um einen zweiten Strahl (B2) fokussierter Strahlung mit Wellenlängen in einem zweiten Wellenlängenbereich mit der zweiten Betriebswellenlänge und einer zweiten numerischen Apparatur (NA2) abzustrahlen, wodurch eine zweite laterale Auflösung ($\Delta x2$) definiert wird, und dahingehend, dass die erste laterale Auflösung ($\Delta x1$) von der zweiten lateralen Auflösung ($\Delta x2$) verschieden ist, und vorzugsweise kleiner als diese ist.

6. System (OCT12) gemäß zumindest einem der Ansprüche 1 bis 5, wobei die erste OCT-Vorrichtung (OCT1) eine Spektralbereichs-OCT-Vorrichtung (SD-OCT) ist und die zweite OCT-Vorrichtung (OCT2) eine Zeitbereichs-OCT-Vorrichtung (TD-OCT) ist.

7. System (OCT12 - OCT12''') gemäß zumindest einem der Ansprüche 1 bis 5, wobei jede der ersten OCT-Vorrichtung (OCT1) und der zweiten OCT-Vorrichtung (OCT2) eine Spektralbereichs-OCT-Vorrichtung (SD-OCT) ist.

8. System (OCT12 - OCT12''') gemäß zumindest einem der Ansprüche 1 bis 7, wobei die erste OCT-Vorrichtung (OCT1) einen ersten Probenarm (SA1) aufweist, der ein erstes Linsensystem (L1) und ein gemeinsames Linsensystem (L12) umfasst, wobei das erste Linsensystem (L1) und das gemeinsame Linsensystem (L12) auf einer ersten optischen Achse angeordnet sind und in Kombination einen ersten fokussierten Abschnitt eines ersten Strahls (B1) in dem ersten Probenarm (SA1) ausbilden, wobei der erste fokussierte Abschnitt eine erste Fokallänge (f) aufweist; dahingehend, dass die zweite OCT-Vorrichtung (OCT2) einen zweiten Probenarm (SA2) aufweist, der ein drittes Linsensystem umfasst, wobei das gemeinsame Linsensystem (L12) und ein spektral teilweise reflektierender Spiegel (M) zwischen dem ersten Linsensystem (L1) und dem gemeinsamen Linsensystem (L12) angeordnet sind, um einen zweiten Strahl (B2), der entlang einer Richtung einer zweiten optischen Achse verläuft, durch das dritte Linsensystem (L3) in die Richtung der ersten optischen Achse und das gemeinsame Linsensystem (L12) durchlaufend umzulenken, wobei das dritte Linsensystem (L3) und das gemeinsame Linsensystem (L12) in Kombination einen zweiten fokussierten Abschnitt des zweiten Strahls in dem zweiten Probenarm (SA2) ausbilden, wobei der zweite fokussierte Abschnitt eine zweite Fokallänge (f2) aufweist;

und dahingehend, dass die erste Fokallänge (f1) von der zweiten Fokallänge (f2) verschieden ist und vorzugsweise kleiner als diese ist.

9. System (OCT12) gemäß Anspruch 8, wobei die erste OCT-Vorrichtung (OCT1) eine erste Lichtquelle (LS1) mit der ersten Betriebswellenlänge ($\lambda$1) und der ersten Bandbreite ($\Delta\lambda$1) umfasst, und die zweite OCT-Vorrichtung (OCT2) eine zweite Lichtquelle (LS2) mit der zweiten Betriebswellenlänge ($\lambda$2) und der zweiten Bandbreite ($\Delta\lambda$2) umfasst, und dahingehend, dass die erste Bandbreite ($\Delta\lambda$1) sich in den Bereich von 100 nm bis 200 nm befindet, und die zweite Bandbreite ($\Delta\lambda$2) kleiner als 20 nm ist.

10. System (OCT12', OCT12") gemäß zumindest einem der Ansprüche 1 bis 7, wobei die erste OCT-Vorrichtung (OCT1) einen ersten Probenarm (SA1) aufweist und die zweite OCT-Vorrichtung (OCT2) einen zweiten Probenarm (SA2) aufweist, der zumindest teilweise dem ersten Probenarm (SA1) räumlich überlagert ist, wobei der erste und der zweite Probenarm (SA1, SA2) durch ein bifokales gemeinsames optisches Linsensystem (BFL12) passieren, das einen ersten Fokussierungsabschnitt (FP1) mit einer ersten Fokallänge (f1), das in dem ersten Probenarm (SA1) wirkt, und einen zweiten Fokussierungsabschnitt (FP2) mit einer zweiten Fokallänge (f2) umfasst, der in dem zweiten Probenarm (SA2) wirkt, und
dahingehend, dass die erste Fokallänge (f1) von der zweiten Fokallänge verschieden ist und vorzugsweise kleiner als diese ist.

11. System (OCT12 - OCT12''') gemäß zumindest einem der Ansprüche 1 oder 10, wobei die erste OCT-Vorrichtung (OCT1) und die zweite OCT-Vorrichtung (OCT2) eine gemeinsame Lichtquelle (LS12) umfassen.

12. System (OCT12") gemäß Anspruch 10 oder 11, wobei die erste OCT-Vorrichtung (OCT1) einen ersten Referenzarm (RA1) umfasst, und die zweite OCT-Vorrichtung (OCT2) einen zweiten Referenzarm (RA2) umfasst, der dem ersten Referenzarm (RA1) zumindest teilweise räumlich überlagert ist,
dahingehend, dass der erste Referenzarm (RA1) eine optische Weglänge im Wesentlichen entsprechend der optischen Weglänge des ersten Probenarms (SA1) aufweist und einen ersten Spiegel (MR1) und ein erstes Referenzarmlängensystem (LR1) umfasst, das einen ersten Referenzarmabschnitt ausbildet, der sich entlang einer ersten Referenzwegrichtung (RAD1) erstreckt und auf den ersten Spiegel (MR1) fokussiert ist, und
dahingehend, dass der zweite Referenzarm (RA2) eine optische Weglänge im Wesentlichen entsprechend der optischen Weglänge des zweiten Probenarms (SA2) aufweist und einen zweiten Spiegel (MR2), einen zweiten teilweise reflektierenden Referenzarmspiegel (MRA), der in dem ersten Referenzarm (RA1) vor dem ersten Referenzarmlinsensystem (LR1) angeordnet ist, und ein zweites Referenzarmlinsensystem (LR2) umfasst, das außerhalb des ersten Referenzarms (RA1) und im Wesentlichen zwischen dem zweiten teilweise reflektierenden Referenzarmspiegel (MRA) und dem zweiten Referenzarmlinsensystem (LR2) angeordnet ist, wobei der teilweise reflektierende Spiegel (MRA) einen Lichtstrahl mit Wellenlängen in einem zweiten Wellenlängenbereich, der mit dem zweiten Referenzarm (RA2) assoziiert ist und durch das erste Referenzarmlinsensystem (LR1) verläuft, in eine zweite Referenzarmrichtung (RAD2) und durch das zweite Referenzarmlinsensystem (LR2) umlenkt, und wobei das erste Referenzarmlinsensystem (LR1) und das zweite Referenzarmlinsensystem (LR2) in Kombination einen zweiten Referenzarmabschnitt ausbildet, der auf den zweiten Spiegel (MR2) fokussiert ist.

13. System (OCT12''') gemäß Anspruch 10 oder 11, wobei die erste OCT-Vorrichtung (OCT1) einen ersten Referenzarm (RA1) umfasst, der durch einen ersten Fokussierungsabschnitt (FPR1) eines bifokalen gemeinsamen Referenzarmlinsensystems (BFLRA) verläuft, und die zweite OCT-Vorrichtung (OCT2) einen zweiten Referenzarm (RA2) umfasst, der dem ersten Referenzarm (RA1) zumindest teilweise räumlich überlagert ist und durch einen zweiten Fokussierungsabschnitt (FPR2) des bifokalen gemeinsamen Referenzarmlinsensystems (BFLRA) passiert,
dahingehend, dass der erste Referenzarm (RA1) weiterhin einen ersten Spiegel (MR1) umfasst, der eingerichtet ist, um Licht mit Wellenlängen in einem ersten Wellenlängenbereich zu reflektieren, der durch die erste Betriebswellenlänge ($\lambda$1) und die erste Bandbreite ($\Delta\lambda$1) definiert ist;
dahingehend, dass der zweite Referenzarm (RA2) weiterhin einen zweiten Spiegel (MR2) umfasst, der eingerichtet ist, um Licht mit Wellenlängen in einem zweiten Längenwellenbereich spektral zu reflektieren, der durch die zweite Betriebswellenlänge und die zweite Bandbreite ($\Delta\lambda$2) definiert ist;
dahingehend, dass eine Fokallänge des ersten Fokussierungsabschnitts (FPR1) derart bemaßt ist, dass die optische Weglänge des ersten Referenzarms (RA1) im Wesentlichen der optischen Weglänge des ersten Probenarms (SA1) entspricht; und
dahingehend, dass eine Fokallänge des zweiten Fokussierungsabschnitts (FPR2) derart bemaßt ist, dass die optische Weglänge des zweiten Referenzarms (RA2) im Wesentlichen der optischen Weglänge des zweiten Probenarms (SA2) entspricht,

wobei vorzugsweise der erste Fokussierungsabschnitt (FPR1) ein kreisförmiger zentraler Abschnitt ist und der zweite Fokussierungsabschnitt (FPR2) ein ringförmiger Abschnitt ist, der den ersten Fokussierungsabschnitt (FPR1) des bifokalen gemeinsamen Referenzarmlinsensystems (BFLRA) umgibt.

14. Verfahren, das ein System gemäß zumindest einem der Ansprüche 1 bis 13 verwendet, zum optischen Messen von internen Abmaßen eines Objekts (10), zum Beispiel eines Auges (20), wobei das Objekt interne Grenzflächen (14, 14', 14") umfasst, bei denen der Brechindex sich derart ändert, dass ein Teil von einfallendem Licht zurückreflektiert und/oder rückgestreut wird und erfasst werden kann, umfassend einen Schritt zum Messen von Abmaßen in einem ersten Teilvolumen (17) des Objekts (10) und von Abmaßen in einer zweiten Teilvolumen (19) des Objekts mittels optischer Kohärenztomographie (OCT) in einer einzigen diagnostischen Sitzung, wobei das zweite Teilvolumen (19) zumindest teilweise von dem ersten Teilvolumen (17) des Objekts (10) verschieden ist.

## Revendications

1. Système (OCT12 - OCT12''') pour la mesure optique de dimensions internes d'un objet-échantillon (10), ledit objet étant un oeil humain (20), ledit objet (10) comprenant des interfaces internes (14, 14', 14") sur lesquelles l'indice de réfraction change de telle sorte qu'une partie d'une lumière incidente est rétroréfléchie et/ou rétrodiffusée et peut être détectée au moyen d'une tomographie par cohérence optique, OCT, comprenant :

   - au moins un premier dispositif d'OCT (OCT1) adapté à mesurer des dimensions internes dans un premier volume partiel (17) de l'objet (10), et
   - au moins un second dispositif d'OCT (OCT2) adapté à mesurer des dimensions internes dans un second volume partiel (19) du même objet (10), dans lequel
   - le second volume partiel (19) est au moins partiellement différent du premier volume partiel (17),

   dans lequel le premier dispositif d'OCT (OCT1) est adapté à émettre un premier faisceau (B1) d'un premier rayonnement ayant des longueurs d'onde dans une première plage de longueurs d'onde définie par une première longueur d'onde opérationnelle ($\lambda$1) et une première bande passante ($\Delta\lambda$1), définissant ainsi une première résolution axiale ;
   dans lequel le second dispositif d'OCT (OCT2) est adapté à émettre un second faisceau (B2) d'un second rayonnement ayant des longueurs d'onde dans une seconde plage de longueurs d'onde définie par une seconde longueur d'onde opérationnelle ($\lambda$2) et une seconde bande passante ($\Delta\lambda$2), définissant ainsi une seconde résolution axiale ;
   dans lequel ce qui suit s'applique :

   - la première longueur d'onde opérationnelle est plus grande que la seconde longueur d'onde opérationnelle, et

   **caractérisé en ce que**
   la première bande passante est considérablement plus grande que la seconde bande passante et
   la première résolution axiale est supérieure à la seconde résolution axiale.

2. Système (OCT12) selon la revendication 1, dans lequel :

   le premier dispositif d'OCT (OCT1) comprend un premier bras de référence (RA1) et un premier bras-échantillon (SA1),
   le second dispositif d'OCT (OCT2) comprend un second bras de référence (RA2) et un second bras-échantillon (SA2), et
   le premier bras-échantillon (SA1) et le second bras-échantillon (SA2) sont superposés spatialement.

3. Système (OCT12 - OCT12''') selon l'une quelconque des revendications précédentes, dans lequel le premier dispositif d'OCT (OCT1) est adapté à mesurer une cornée (22) et une section antérieure (24) dudit oeil (20) et/ou en ce que le second dispositif d'OCT (OCT2) est adapté à mesurer une longueur, par ex. mesurée le long d'un sens de la profondeur, et/ou la rétine (26) dudit oeil (20).

4. Système (OCT12, OCT12') selon l'une quelconque des revendications précédentes, dans lequel le premier dispositif d'OCT (OCT1) est adapté à émettre un premier faisceau (B1) focalisé avec une première longueur focale prédéterminée (f1) et en ce que le second dispositif d'OCT (OCT2) est adapté à émettre un second faisceau (B2) focalisé avec une seconde longueur focale prédéterminée (f2), dans lequel la première longueur focale (f1) est plus courte que la seconde longueur focale (f2).

5. Système (OCT12 - OCT12''') selon l'une quelconque des revendications précédentes, dans lequel le premier dispositif d'OCT (OCT1) est adapté à émettre un premier faisceau (B1) de rayonnement focalisé présentant des longueurs d'onde dans une première plage de longueur d'onde ayant la première longueur d'onde opérationnelle ($\lambda$1) et une première ouverture numérique (NA1), définissant ainsi une première résolution latérale ($\Delta$X1) ;

en ce que le second dispositif d'OCT (OCT2) est adapté à émettre un second faisceau (B2) de rayonnement focalisé présentant des longueurs d'onde dans une seconde plage de longueur d'onde ayant la seconde longueur d'onde opérationnelle et une seconde ouverture numérique (NA12), définissant ainsi une seconde résolution latérale ($\Delta$X2), et

en ce que la première résolution latérale ($\Delta$X1) est différente de, de préférence plus petite que, la seconde résolution latérale ($\Delta$X2).

6. Système (OCT12) selon l'une quelconque des revendications 1 à 5, dans lequel le premier dispositif d'OCT (OCT1) est un dispositif d'OCT à domaine spectral (SD-OCT) et le second dispositif OCT (OCT2) est un dispositif d'OCT à domaine temporel (TD-OCT).

7. Système (OCT12 - OCT12''') selon l'une quelconque des revendications 1 à 5, dans lequel chacun des premier dispositif d'OCT (OCT1) et second dispositif d'OCT (OCT2) est un dispositif d'OCT à domaine spectral (SD-OCT).

8. Système (OCT12 - OCT12''') selon l'une quelconque des revendications 1 à 7, dans lequel le premier dispositif d'OCT (OCT1) présente un premier bras-échantillon (SA1) comprenant un premier système de lentilles (L1) et un système de lentilles communes (L12), dans lequel le premier système de lentilles (L1) et le système de lentilles communes (L12) sont agencés sur un premier axe optique et forment en combinaison une première partie focalisée d'un premier faisceau (B1) dans le premier bras-échantillon (SA1), la première partie focalisée présentant une première longueur focale (f1) ;

en ce que le second dispositif d'OCT (OCT2) présente un second bras-échantillon (SA2) comprenant un troisième système de lentilles (L3), ledit système de lentilles communes (L12) et un miroir partiellement réfléchissant au niveau du spectre (M) agencé entre le premier système de lentilles (L1) et le système de lentilles communes (L12) de façon à rediriger un second faisceau (B2) passant le long d'un sens d'un second axe optique à travers le troisième système de lentilles (L3) dans le sens du premier axe optique et passant à travers ledit système de lentilles communes (L12), dans lequel le troisième système de lentilles (L3) et le système de lentilles communes (L12) forment en combinaison une seconde partie focalisée du second faisceau dans le second bras-échantillon (SA2), la seconde partie focalisée présentant une seconde longueur focale (f2) ;

et en ce que la première longueur focale (f1) est différente de, de préférence plus petite que, la seconde longueur focale (f2).

9. Système (OCT12) selon la revendication 8, dans lequel le premier dispositif d'OCT (OCT1) comprend une première source lumineuse (LS1) présentant la première longueur d'onde opérationnelle ($\lambda$1) et la première bande passante ($\Delta\lambda$1), et le second dispositif d'OCT (OCT2) comprend une seconde source lumineuse (LS2) présentant la seconde longueur d'onde opérationnelle ($\lambda$2) et la seconde bande passante ($\Delta\lambda$2),

et en ce que la première bande passante ($\Delta\lambda$1) est située dans la plage de 100 nm à 200 nm, et la seconde bande passante ($\Delta\lambda$2) est inférieure à 20 nm.

10. Système (OCT12', OCT12'') selon l'une quelconque des revendications 1 à 7, dans lequel le premier dispositif d'OCT (OCT1) présente un premier bras-échantillon (SA1) et le second dispositif d'OCT (OCT2) présente un second bras-échantillon (SA2) au moins partiellement superposé spatialement sur le premier bras-échantillon (SA1), les premier et second bras-échantillons (SA1, SA2) passent à travers un système de lentilles optiques communes bifocales (BFL12) comprenant une première partie de mise au point (FP1) présentant une première longueur focale (f1) et agissant dans le premier bras-échantillon (SA1), et une seconde partie de mise au point (FP2) présentant une seconde longueur focale (f2) et agissant dans le second bras-échantillon (SA2), et

en ce que la première longueur focale (f1) est différente de, de préférence plus petite que, la seconde longueur focale (f2).

11. Système (OCT12 - OCT12''') selon l'une des revendications 1 ou 10, dans lequel le premier dispositif d'OCT (OCT1) et le second dispositif d'OCT (OCT2) comprennent une source lumineuse commune (LS12).

12. Système (OCT12'') selon la revendication 10 ou 11, dans lequel le premier dispositif d'OCT (OCT1) comprend un premier bras de référence (RA1) et le second dispositif d'OCT (OCT2) comprend un second bras de référence (RA2) qui est au moins partiellement superposé spatialement sur le premier bras de référence (RA1),

en ce que le premier bras de référence (RA1) présente une longueur de trajet optique correspondant essentiellement à la longueur de trajet optique du premier bras-échantillon (SA1) et comprend un premier miroir (MR1) et un premier système de longueur de bras de référence (LR1) formant une première partie de bras de référence qui s'étend le long d'une première direction de trajet de référence (RAD1) et focalisée sur le premier miroir (MR1), et

en ce que le second bras de référence (RA2) comprend une longueur de trajet optique correspondant essentiellement à la longueur de trajet optique du second bras-échantillon (SA2) et comprend un second miroir (MR2), un miroir réfléchissant partiellement (MRA) le second bras de référence, qui est agencé dans le premier bras de référence (RA1) devant le premier système de lentilles de bras de référence (LR1), et un second système de lentilles de bras de référence (LR2), qui est agencé en-dehors du premier bras de référence (RA1) et sensiblement entre le miroir réfléchissant partiellement (MRA) le second bras de référence et le second système de lentilles de bras de référence (LR2), dans lequel que le miroir réfléchissant partiellement (MRA) redirige un faisceau de lumière ayant une longueur d'onde dans une seconde plage de longueurs d'onde associée au second bras de référence (RA2) et passant à travers le premier système de lentilles de bras de référence (LR1) dans une seconde direction de bras de référence (RAD2) et à travers le second système de lentilles de bras de référence (LR2), et dans lequel le premier système de lentilles de bras de référence (LR1) et le second système de lentilles de bras de référence (LR2) forment en combinaison une seconde partie de bras de référence qui est focalisée sur le second miroir (MR2).

13. Système (OCT12''') selon la revendication 10 ou 11, dans lequel le premier dispositif d'OCT (OCT1) comprend un premier bras de référence (RA1) passant à travers une première partie de mise au point (FPR1) d'un système de lentilles bifocales communes de bras de référence (BFLRA) et le second dispositif d'OCT (OCT2) comprend un second bras de référence (RA2) qui est au moins partiellement superposé spatialement sur le premier bras de référence (RA1) et passe à travers une seconde partie de mise au point (FPR2) du système de lentilles bifocales communes de bras de référence (BFLRA),

en ce que le premier bras de référence (RA1) comprend en outre un premier miroir (MR1) adapté à réfléchir une lumière ayant des longueurs d'onde dans une première plage de longueurs d'onde définie par la première longueur d'onde opérationnelle ($\lambda$1) et la première bande passante ($\Delta\lambda$1) ;

en ce que le second bras de référence (RA2) comprend en outre un second miroir (MR2) adapté à réfléchir de façon spectrale une lumière ayant des longueurs d'onde dans une seconde plage de longueurs d'onde définie par la seconde longueur d'onde opérationnelle ($\lambda$2) et la second bande passante ($\Delta\lambda$2) ;

en ce qu'une longueur focale de la première partie de mise au point (FPR1) est dimensionnée de telle sorte que la longueur de trajet optique du premier bras de référence (RA1) correspond sensiblement à la longueur de trajet optique du premier bras-échantillon (SA1) ; et

en ce qu'une longueur focale de la seconde partie de mise au point (FPR2) est dimensionnée de telle sorte que la longueur de trajet optique du second bras de référence (RA2) correspond sensiblement à la longueur de trajet optique du second bras-échantillon (SA2),

dans lequel, de préférence, la première partie de mise au point (FPR1) est une partie centrale circulaire et la seconde partie de mise au point (FPR2) est une partie annulaire entourant la première partie de mise au point (FPR1) du système de lentilles bifocales communes de bras de référence (BFLRA).

14. Procédé employant un système selon l'une quelconque des revendications 1 à 13 pour la mesure optique de dimensions internes d'un objet (10), par exemple un oeil (20), ledit objet (10) comprenant des interfaces internes (14, 14', 14") sur lesquelles l'indice de réfraction change de telle sorte qu'une partie d'une lumière incidente est rétroréfléchie et/ou rétrodiffusée et peut être détectée, comprenant une étape de mesure de dimensions dans un premier volume partiel (17) de l'objet (10) et de dimensions dans un second volume partiel (19) de l'objet au moyen d'une tomographie par cohérence optique (OCT) dans une recherche de diagnostic unique, dans lequel le second volume partiel (19) est au moins partiellement différent du premier volume partiel (17) de l'objet (10).

FIG 1 (PRIOR ART)

FIG 2 (PRIOR ART)

FIG 3

FIG 4

FIG 5

for example: $\lambda_2 = 700\ nm$     $\lambda_1 = 850\ nm$

$\Delta\lambda_2 = 20\ nm$     $\Delta\lambda_1 = 200\ nm$

$(750 \leqslant \lambda_1 \leqslant 950\ nm)$

FIG 6

FIG 7

SD-OCT

OCT12'''

LS12

SOF12

B12

L1

BFLRA

FPR1

DOF12

BS12

FPR2

DL1

SA12

B12'

RA1

RA2

DG12

FP2 FP1

RA12

MR1

MR2

DL2

SDA12

BFL12

Δλ2

B1

B2

λ2

16

17

14

λ1

17, DOF1

Δλ1

14'

19, DOF2

12

10

B1

FFT12

COMP12

14''

OCT12'''

18

B2

19

EP 2 675 338 B1

FIG 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010134564 A1

### Non-patent literature cited in the description

- **I. GRULKOWSKI et al.** Anterior segment imaging with Spectral OCT system using a high-speed CMOS camera. *OPTICS EXPRESS,* 12 March 2009, vol. 17 (6), 4842
- **J. JUNGWIRTH et al.** Extended in vivo anterior eye-segment imaging with full-range complex spectral domain optical coherence tomography. *Journal of Biomedical Optics Letters,* September 2009, vol. 14 (5
- **R.J. ZAWADZKI et al.** Three-dimensional ophthalmic optical coherence tomography with a refraction correction algorithm. *SPIE proceedings,* vol. 5140
- **DR. H.P. ISELI et al.** Iterative Berechnung von Ablationsprofilen in der Refraktiven Chirurgie. *Augenspiegel,* 2008, vol. 20, 07-08